# EUROPEAN PATENT SPECIFICATION

(11) **EP 3 309 141 B1**
(45) Date of publication and mention of the grant of the patent: **01.09.2021**
(21) Application number: 16807450.8
(22) Date of filing: 07.06.2016
(51) Int. Cl.: C07C 211/54, C07C 211/61, C07D 209/88, C07D 307/91, C09K 11/06, H01L 51/50

(54) **ARYLAMINE COMPOUND AND ORGANIC ELECTROLUMINESCENT DEVICE**
ARYLAMINVERBINDUNG UND ORGANISCHES ELEKTROLUMINESZENZELEMENT
COMPOSÉ ARYLAMINE ET ÉLÉMENT ÉLECTROLUMINESCENT ORGANIQUE

(30) Priority: 11.06.2015 JP 2015118366
(43) Date of publication of application: 18.04.2018
(73) Proprietor: Hodogaya Chemical Co., Ltd., Chuo-ku Tokyo 104-0028 (JP)
(72) Inventor: OOKUMA, Hiroshi, Tokyo 104-0028 (JP); NAGAOKA, Makoto, Tokyo 104-0028 (JP); SURUGA, Kazuyuki, Tokyo 104-0028 (JP)
(74) Representative: Müller-Boré & Partner Patentanwälte PartG mbB
(86) International application number: PCT/JP2016/066838
(87) International publication number: WO 2016/199743

(56) References cited:
- WO-A1-2006/077130
- WO-A1-2014/017042
- WO-A1-2014/073791
- WO-A1-2014/073791
- WO-A1-2014/088047
- WO-A1-2014/088047
- WO-A1-2014/189072
- WO-A1-2014/189073
- WO-A1-2014/200260
- WO-A1-2014/200260
- WO-A1-2015/080183
- WO-A1-2015/111888
- WO-A1-2015/126090
- WO-A1-2015/130069
- JP-A- 2012 532 111
- JP-A- 2013 526 548
- JP-A- 2015 220 298
- JP-A- 2016 100 364
- KR-A- 20110 032 238
- US-A1- 2011 278 556
- US-A1- 2013 256 649
- CHRISTOPH LAMBERT ET AL: "Charge-Transfer Interactions in a Multichromophoric Hexaarylbenzene Containing Pyrene and Triarylamines", JOURNAL OF ORGANIC CHEMISTRY, vol. 77, no. 14, 20 July 2012 (2012-07-20) , pages 6147-6154, XP55530589, ISSN: 0022-3263, DOI: 10.1021/jo300924x
- WATANABE SOICHI ET AL: "Hexaphenylbenzene derivatives for blue organic light-emitting devices", CHEMISTRY LET, CHEMICAL SOCIETY OF JAPAN, JAPAN, vol. 36, no. 5, 1 January 2007 (2007-01-01), pages 590-591, XP009100323, ISSN: 0366-7022, DOI: 10.1246/CL.2007.590
- THOMAS K R J ET AL: "Hexaphenylphenylene dendronized pyrenylamines for efficient organic light-emitting diodes", JOURNAL OF MATERIALS CHEMISTRY, ROYAL SOCIETY OF CHEMISTRY, GB, vol. 15, no. 41, 1 January 2005 (2005-01-01), pages 4453-4459, XP003019890, ISSN: 0959-9428, DOI: 10.1039/B509325B
- GAGNON ERIC ET AL: "Triarylamines Designed to Form Molecular Glasses. Derivatives of Tris( p -terphenyl-4-yl)amine with Multiple Contiguous Phenyl Substituents", ORGANIC LETTERS, vol. 12, no. 3, 5 February 2010 (2010-02-05), pages 404-407, XP55783418, US ISSN: 1523-7060, DOI: 10.1021/ol902841c

## Description

### Technical Field

This invention relates to a compound suitable for an organic electroluminescent device which is a self light-emitting device preferred for various displays; and this device. In detail, the invention relates to an arylamine compound, and an organic electroluminescent device (may hereinafter be referred to as an organic EL device) using the compound.

### Background Art

Since an organic EL device is a self light-emitting device, it is brighter, better in visibility, and capable of clearer display, than a liquid crystal device. Hence, energetic researches have been conducted on organic EL devices.

In 1987, C.W. Tang et al. of Eastman Kodak developed a laminated structure device sharing various roles among different materials, thereby imparting practical applicability to organic EL devices using organic materials. Such an organic EL device is formed by laminating a layer of tris(8-hydroxyquinoline)aluminum (Alq₃), which is a fluorescent body capable of transporting electrons, and a layer of an aromatic amine compound capable of transporting holes. Because of this configuration, the organic EL device injected positive charges and negative charges into the layer of the fluorescent body to perform light emission, thereby obtaining a high luminance of 1,000 cd/m² or more at a voltage of 10V or less.

Many improvements have been heretofore made to put the organic EL devices to practical use. For example, high efficiency and high durability have been achieved by an electroluminescent device, in which the roles of the respective layers in a laminated structure are shared among more types of materials, and an anode, a hole injection layer, a hole transport layer, a luminous layer, an electron transport layer, an electron injection layer, and a cathode are provided on a substrate.

For a further increase in the luminous efficiency, it has been attempted to utilize triplet excitons, and the utilization of phosphorescent compounds has been considered. Furthermore, devices utilizing light emission by thermally activated delayed fluorescence (TADF) have been developed. Adachi et al. of Kyushu University realized in 2011 an external quantum efficiency of 5.3% by a device using a thermally activated delayed fluorescence material.

The luminous layer can also be prepared by doping a charge transporting compound, generally called a host material, with a fluorescent compound, a phosphorescent compound, or a material radiating delayed fluorescence. The selection of the organic material in the organic EL device greatly affects the characteristics of the device, such as efficiency and durability.

With the organic EL device, the charges injected from both electrodes recombine in the luminous layer to obtain light emission. For this purpose, how efficiently the both charges of the holes and the electrons are passed on to the luminous layer is of importance in the organic EL device, and the device needs to be excellent in carrier balance. Moreover, the hole injecting properties are enhanced, and the electron blocking properties of blocking electrons injected from the cathode are enhanced, whereby the probability of the holes and the electrons recombining is increased. Besides, excitons generated within the luminous layer are confined. By so doing, a high luminous efficiency can be obtained. Thus, the role of the hole transport material is so important that there has been a desire for a hole transport material having high hole injection properties, allowing marked hole mobility, possessing high electron blocking properties, and having high durability to electrons.

From the viewpoint of device lifetime, heat resistance and amorphousness of the material are also important. A material with low heat resistance is thermally decomposed even at a low temperature by heat produced during device driving, and the material deteriorates. With a material having low amorphousness, crystallization of a thin film occurs even in a short time, and the device deteriorates. Thus, high resistance to heat and satisfactory amorphousness are required of the material to be used.

As hole transport materials so far used for organic EL devices, N,N'-diphenyl-N,N'-di(α-naphthyl)benzidine (NPD) and various aromatic amine derivatives have been present (see Patent Documents 1 and 2). NPD has satisfactory hole transport capability, but its glass transition temperature (Tg) serving as an index of heat resistance is as low as 96°C. Under high temperature conditions, moreover, it causes decline in device characteristics due to crystallization.

Among the aromatic amine derivatives described in Patent Documents 1 and 2 are compounds having excellent hole mobility of 10⁻³ cm²/Vs or more. Since the electron blocking properties of such aromatic amine derivatives are insufficient, however, some of electrons pass through the luminous layer, and an increase in the luminous efficiency cannot be expected. Thus, there has been a desire for a material having higher electron blocking properties, more stable in the form of a thin film, and possessing higher resistance to heat, in order to achieve an even higher efficiency.

As compounds improved in characteristics such as heat resistance and hole injection properties, arylamine compounds having substituted carbazole structures have been proposed in Patent Documents 3 and 4. In devices using these compounds as hole injection layers or hole transport layers, heat resistance and luminous efficiency have been improved. However, the improved characteristics have been still insufficient, and an even lower driving voltage and an even higher luminous efficiency are desired.

### Prior Art Documents

### Patent Documents

Patent Document 1: JP-A-H-8-48656
Patent Document 2: Japanese Patent No. 3194657
Patent Document 3: JP-A-2006-151979
Patent Document 4: WO2008/62636 WO2014088047 also discloses similar compounds for use as organic electroluminesce element.

### Summary of the Invention

### Problems to be solved by the invention

It is an object of the present invention to provide an organic compound, as a material for an organic EL device, (1) which is excellent in hole injection properties, (2) which excels in hole transport performance, (3) which has electron blocking capability, (4) which is highly stable in a thin film state, and (5) which is excellent in heat resistance.

It is another object of the present invention to provide an organic EL device, by use of this compound, (1) which is high in luminous efficiency and power efficiency, (2) which is low in practical driving voltage, and (3) which has a long lifetime.

### Means for solving the problems

In an attempt to attain the above objects, the present inventors paid attention to the fact that an aromatic tertiary amine structure had high hole injection/transport capabilities. They also held expectations for the heat resistance and thin-film stability of the aromatic tertiary amine structure. Against the background of these circumstances, the inventors designed and chemically synthesized a monoamine compound having a novel triarylamine structure. Using this compound, they prototyped various organic EL devices, and energetically evaluated their device characteristics. As a result, they have accomplished the present invention.
1) The present invention is an arylamine compound represented by the following general formula (1): 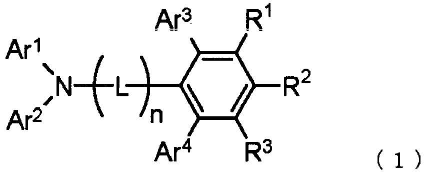 where
   Ar¹ and Ar² may be the same or different and each represent a phenyl group having a substituent or a fluorenyl group having a substituent.Ar³ and Ar⁴ represent an unsubstituted phenyl group,
   n denotes an integer of 1 to 2,
   L represents a 1,4-phenylene group,
   R¹ and R³ each represent a hydrogen atom; R² is an unsubstituted phenyl group,

According to the present invention, moreover, there is provided an organic EL device having a pair of electrodes and at least one organic layer sandwiched therebetween, wherein the arylamine compound is used for the organic layer.

In the organic EL device of the present invention, it is preferred for the organic layer to be a hole transport layer, an electron blocking layer, a hole injection layer, or a luminous layer.

### Effects of the invention

The arylamine compound of the present invention is a novel compound and, compared with conventional hole transport materials, has high hole mobility, has excellent electron blocking capability, is stable in a thin film state, and is excellent in heat resistance.

The arylamine compound of the present invention, as compared with conventional materials, is high in hole injection properties, has high hole mobility, is high in electron blocking properties, and is highly stable to electrons. In an organic EL device using the arylamine compound of the present invention as a constituent material of a hole injection layer and/or a hole transport layer, therefore, excitons generated within a luminous layer can be confined, and the probability of holes and electrons recombining can be increased, and a high luminous efficiency can be obtained. Furthermore, the driving voltage is lowered, and the durability is enhanced.

The arylamine compound of the present invention has excellent electron blocking properties, is better in hole transport properties in comparison with conventional materials, and is highly stable in a thin film state. Thus, an organic EL device using the arylamine compound of the present invention as a constituent material of an electron blocking layer has a high luminous efficiency, but is lowered in driving voltage and improved in current resistance, so that maximum brightness is increased.

The arylamine compound of the present invention is better in hole transport properties and wider in bandgap than conventional materials. Thus, when the arylamine compound of the present invention is used as a host material, and a fluorescent luminous body, a phosphorescent luminous body or a material radiating delayed fluorescence, called a dopant, is carried by the host material to form a luminous layer, an organic EL device lowered in driving voltage and improved in luminous efficiency can be realized.

As described above, the arylamine compound of the present invention is useful as a material constituting the hole injection layer, hole transport layer, electron blocking layer, or luminous layer of an organic EL device. The organic EL device of the present invention is high in luminous efficiency and power efficiency, and can thus lower the practical driving voltage of the device. Besides, the light emission start voltage can be lowered, and the durability can be improved.

### Brief Description of the Drawings

[Fig. 1] is a ¹H-NMR chart of Compound 2 of Example 1.
[Fig. 2] is a ¹H-NMR chart of Compound 10 of Example 2.
[Fig. 3] is a ¹H-NMR chart of Compound 41 of Example 3.
[Fig. 4] is a ¹H-NMR chart of Compound 42 of Example 4.
[Fig. 5] is a ¹H-NMR chart of Compound 57 of Example 5.
[Fig. 6] is a ¹H-NMR chart of Compound 62 of Example 6.
[Fig. 7] is a ¹H-NMR chart of Compound 91 of Example 7.
[Fig. 8] is a ¹H-NMR chart of Compound 92 of Example 8.
[Fig. 9] is a ¹H-NMR chart of Compound 70 of Example 9.
[Fig. 10] is a ¹H-NMR chart of Compound 94 of Example 10.
[Fig. 11] is a view showing the EL device configuration of Device Examples and Device Comparative Examples.
[Fig. 12] is a view showing Compounds 1 to 10 which are arylamine compounds according to the present invention.
[Fig. 13] is a view showing Compounds 11 to 18 which are arylamine compounds according to the present invention.
[Fig. 14] is a view showing Compounds 19 to 26 which are arylamine compounds according to the present invention.
[Fig. 15] is a view showing Compounds 27 to 34 which are arylamine compounds according to the present invention.
[Fig. 16] is a view showing Compounds 35 to 44 which are arylamine compounds according to the present invention.
[Fig. 17] is a view showing Compounds 45 to 54 which are arylamine compounds according to the present invention.
[Fig. 18] is a view showing Compounds 55 to 64 which are arylamine compounds according to the present invention.
[Fig. 19] is a view showing Compounds 65 to 74 which are arylamine compounds according to the present invention.
[Fig. 20] is a view showing Compounds 75 to 82 which are arylamine compounds according to the present invention.
[Fig. 21] is a view showing Compounds 83 to 92 which are arylamine compounds according to the present invention.
[Fig. 22] is a view showing Compounds 93 to 95 which are arylamine compounds according to the present invention.

### Mode for Carrying Out the Invention

The arylamine compound of the present invention is a novel compound, and is represented by the following general formula (1) :

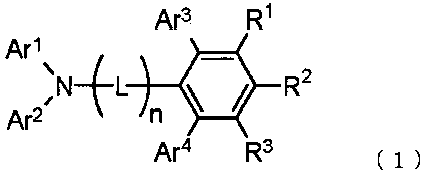

Ar¹ and Ar² may be the same or different, and each represent, a phenyl group, or a fluorenyl group; particularly preferably a substituted phenyl group, or a substituted fluorenyl group. As the substituent for the phenyl group, a phenyl group, a biphenylyl group, a terphenylyl group, a naphthyl group, a phenanthrenyl group, or a fluorenyl group is preferred. As the substituent for the fluorenyl group, a methyl group or a phenyl group is preferred.

From the viewpoint of synthesis, if Ar¹ or Ar² is substituted, Ar¹ or Ar² being substituted preferably has a structure different from a structure composed of a benzene ring, and L, Ar³, Ar⁴ and R¹ to R³ bound to the benzene ring.

Ar³ and Ar⁴ are each a phenyl group (unsubstituted). L is a 1,4-phenylene group.

As n, is 1 or 2, 1 is preferred from the viewpoint of synthesis.

R¹ and R³ each represent a hydrogen atom.

R² is an unsubstituted phenyl group,

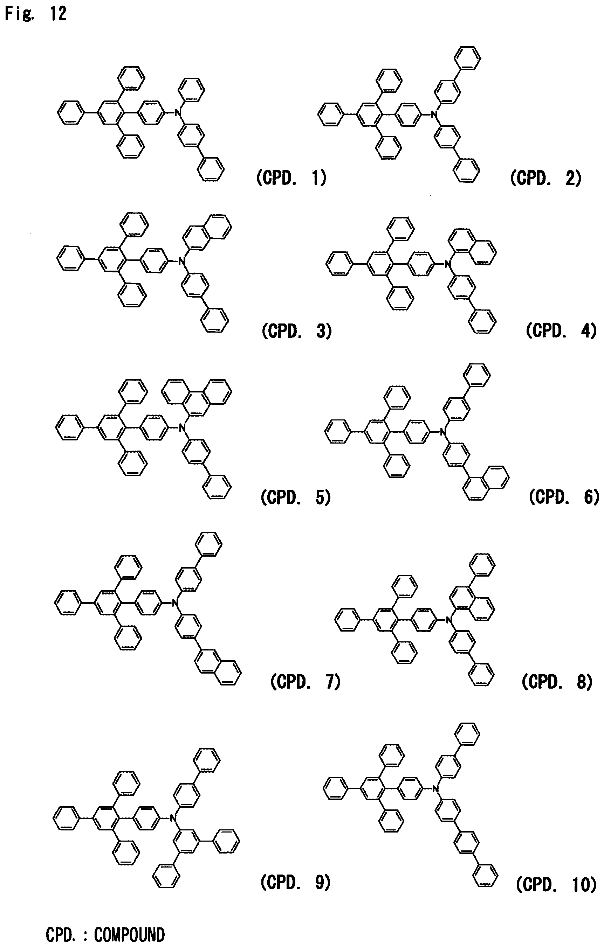

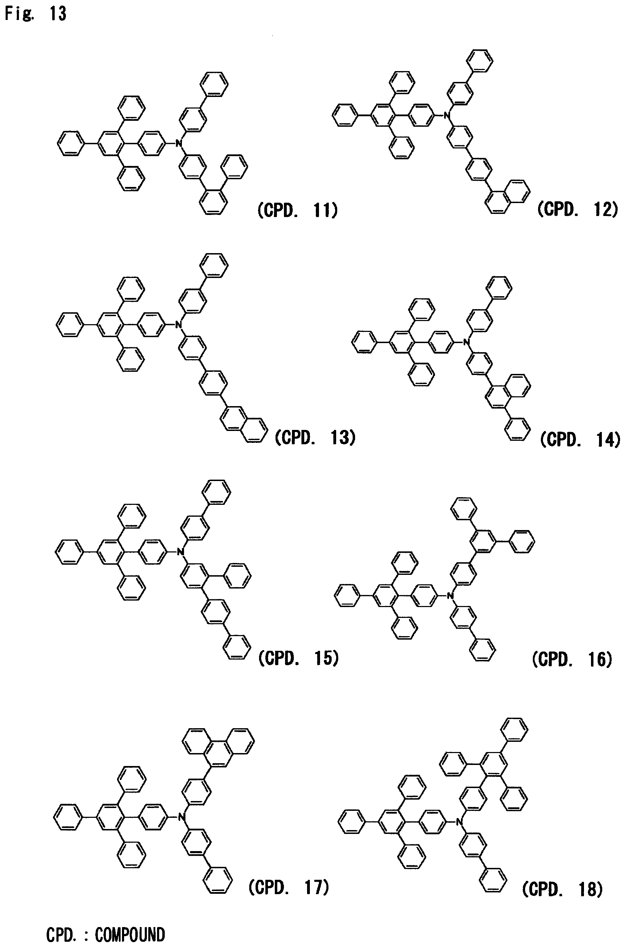

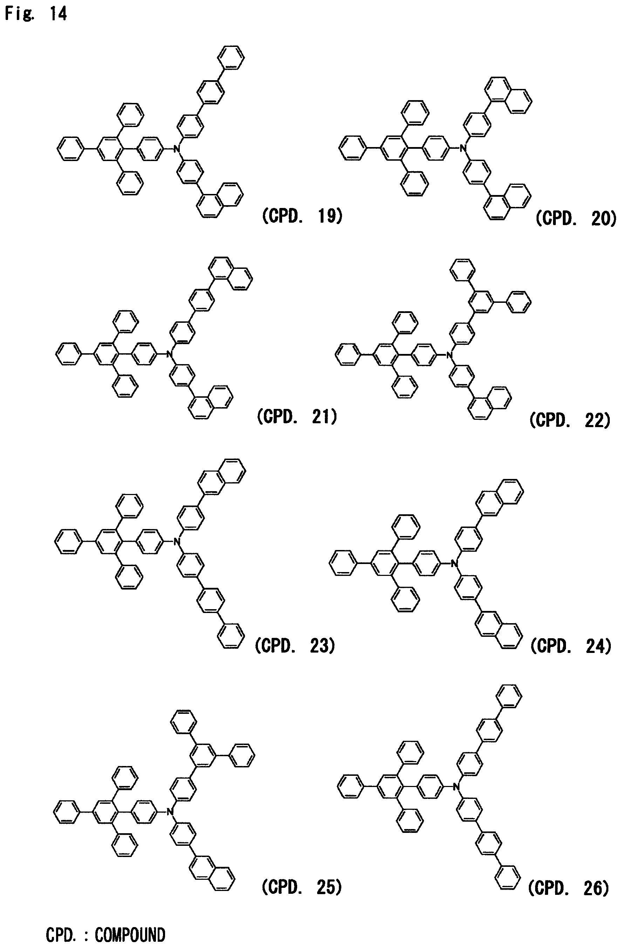

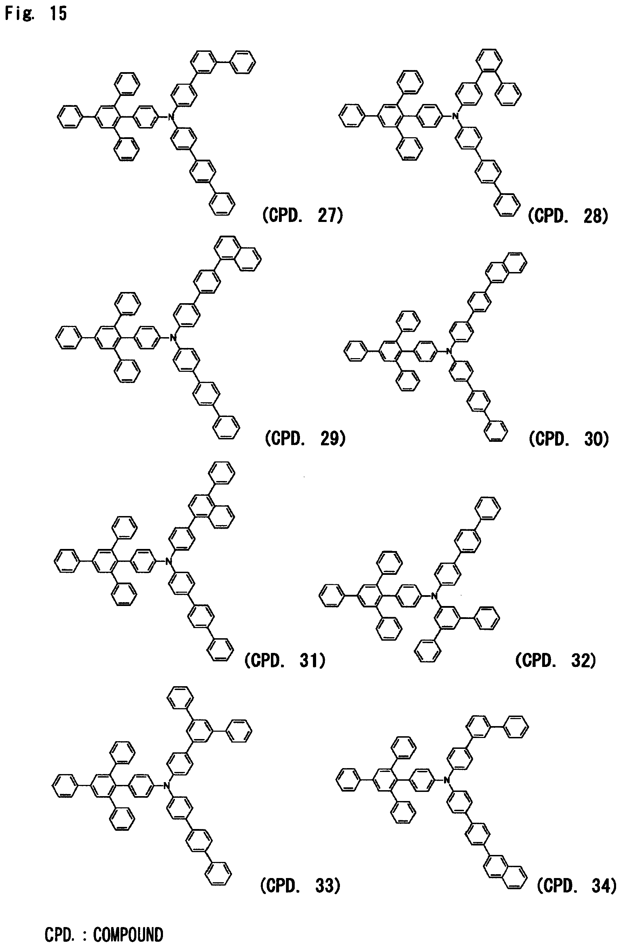

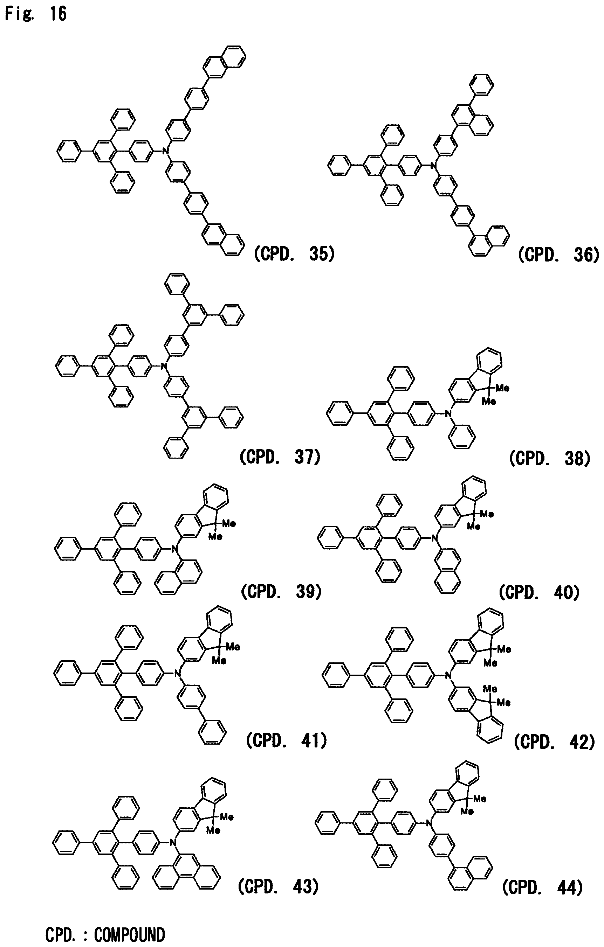

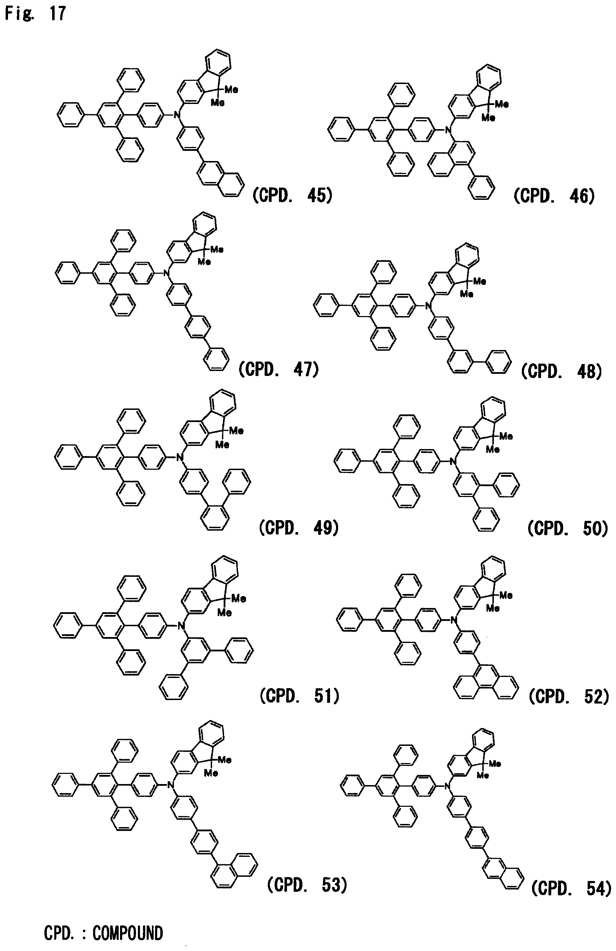

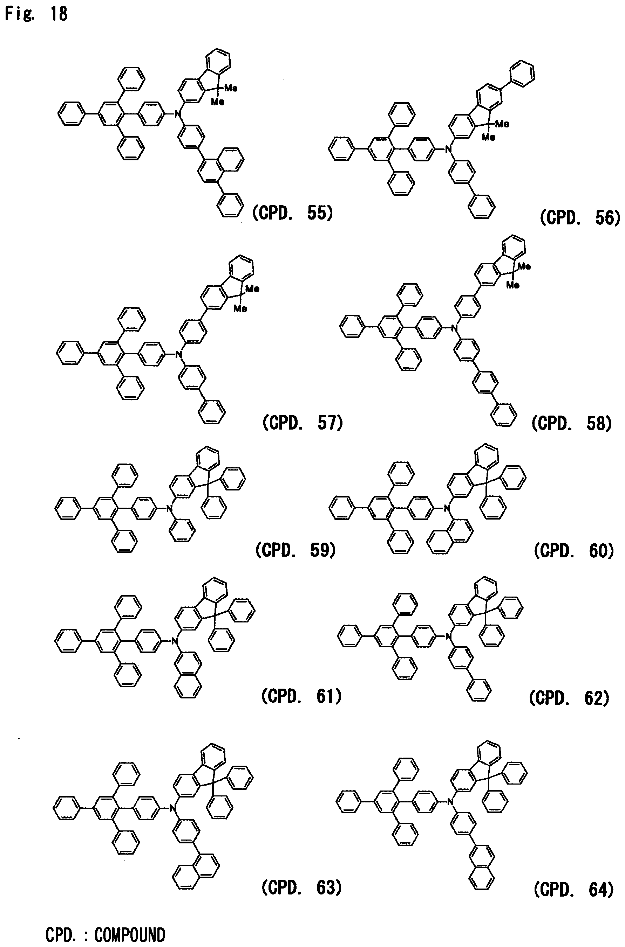

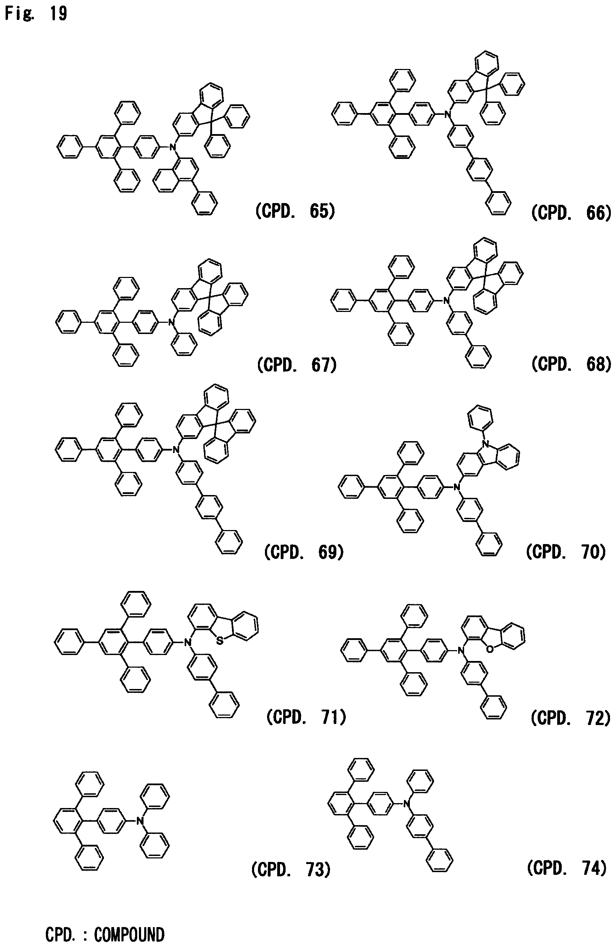

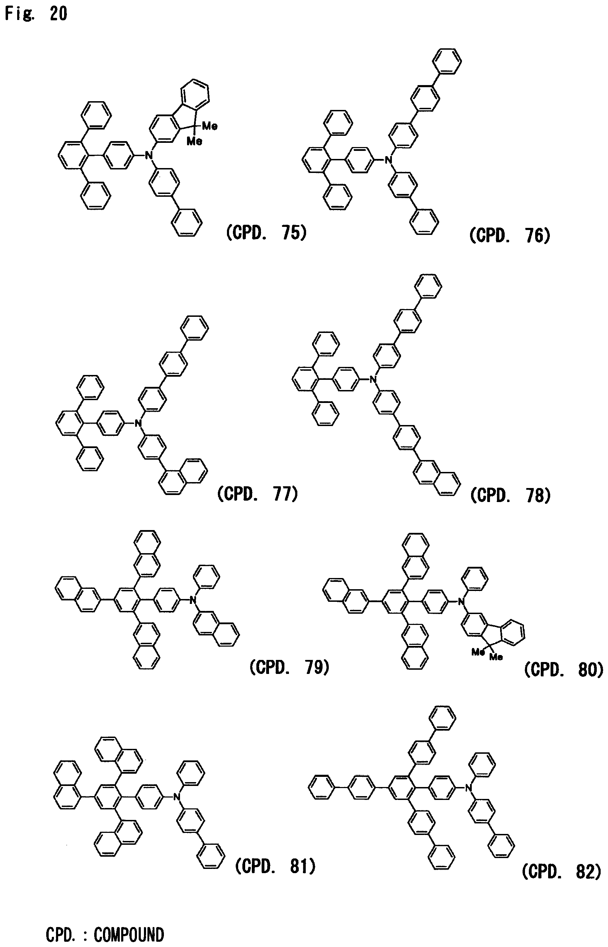

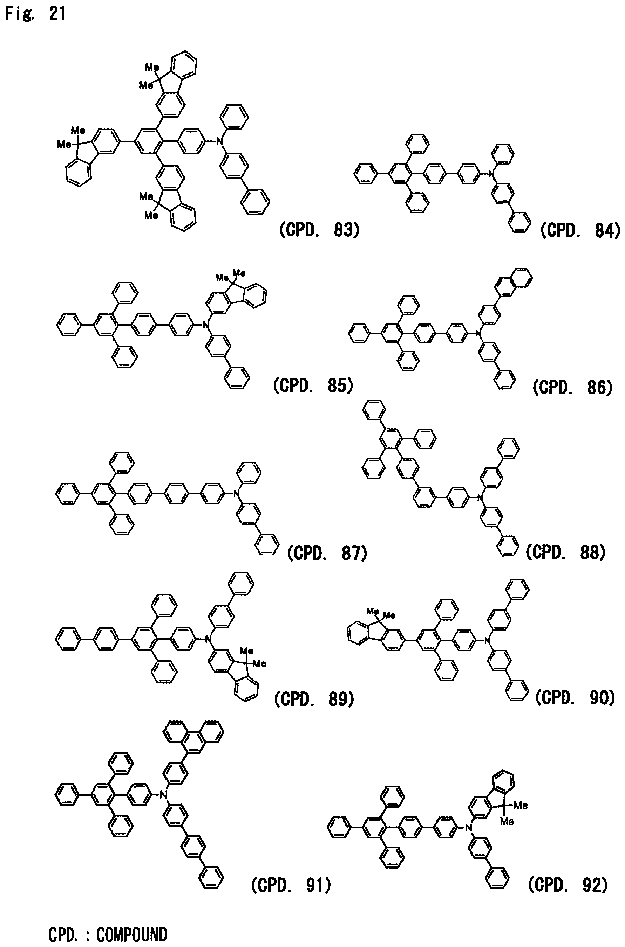

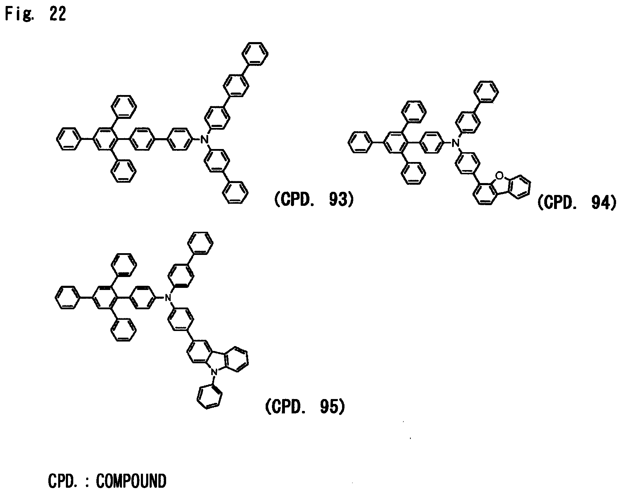

Of the arylamine compounds according to the present invention, concrete examples of the preferred compound are shown in Figs. 12 to 22, but the arylamine compounds of the present invention are not limited to these compounds. "Me" in the structural formulas stands for a methyl group.

Of the exemplary compounds shown in Figs. 12 to 22, Compounds 84 to 86, 92 and 93 correspond to the compound of the formula (1) in which n=2, and Compounds 87 and 88 correspond to the compound of the formula (1) in which n=3.

### <Manufacturing method>

The arylamine compound of the present invention is a novel compound and, for example, can be synthesized in the following manner:
Using any primary or secondary aromatic amine derivative and any aromatic halide, a known cross coupling reaction such as Ullmann coupling or Suzuki coupling is performed to synthesize a secondary or tertiary aromatic amine derivative.

The resulting secondary or tertiary aromatic amine derivative is halogenated by a known method to obtain a halogeno-secondary amine derivative or a halogeno-tertiary amine derivative (they are collectively called halides).

The resulting halide is reacted with pinacolborane, bis (pinacolato) diboron, trimethyl borate or the like, whereby boronic acid or a boronic acid ester derivative can be synthesized.

Separately, any aromatic hydrocarbon compound (for example, 1, 3, 5-triphenylbenzene derivative) is halogenated by a known method to obtain a halogeno-aromatic hydrocarbon compound.

Using the resulting halogeno-aromatic hydrocarbon compound and the above boronic acid or the boronic acid ester derivative, a known cross coupling reaction such as Suzuki coupling is performed, whereby a targeted arylamine compound can be synthesized.

The foregoing manufacturing method is an example, and the desired arylamine compound can be obtained otherwise, for example, by combining the known cross coupling reaction and halogenation reaction, as appropriate, as in Examples 7 to 10.

The purification of the resulting compound can be performed, for example, by purification using a column chromatograph, by adsorption purification using silica gel, activated carbon, activated clay, or the like, by recrystallization or crystallization using a solvent, or by sublimation purification. The identification of the compounds can be made by NMR analysis. As the physical properties, the glass transition temperature (Tg) and the work function can be measured.

The glass transition temperature (Tg) serves as an index to stability in a thin film state. The glass transition temperature (Tg) can be measured by a high sensitivity differential scanning calorimeter (DSC3100S, produced by Bruker AXS) using a powder.

The work function serves as an index to hole transport properties. The work function can be measured by preparing a 100 nm thin film on an ITO substrate, and making a measurement using an ionization potential measuring device (PYS-202, produced by Sumitomo Heavy Industries, Ltd.).

### <Organic EL device>

The arylamine compound of the present invention is suitable as a material for an organic layer in an organic EL device. An organic EL device formed using the arylamine compound of the present invention (may hereinafter be referred to as the organic EL device of the present invention), for example, has a structure in which an anode, a hole injection layer, a hole transport layer, a luminous layer, an electron transport layer, and a cathode are formed in this sequence on a substrate such as a glass substrate or a transparent plastic substrate (e.g., polyethylene terephthalate substrate).

The organic EL device of the present invention may further have an electron blocking layer between the hole transport layer and the luminous layer. A hole blocking layer may be provided between the luminous layer and the electron transport layer. An electron injection layer may be provided between the electron transport layer and the cathode.

In the organic EL device of the present invention, some of the organic layers can be omitted, or can be allowed to concurrently serve as the other layers. For example, a layer concurrently serving as the hole injection layer and the hole transport layer can be formed, or a layer concurrently serving as the electron injection layer and the electron transport layer can be formed. Furthermore, a constitution in which two or more of the organic layers having the same function are laminated can be adopted. It is also possible to adopt a constitution in which two of the hole transport layers are laminated, a constitution in which two of the luminous layers are laminated, or a constitution in which two of the electron transport layers are laminated.

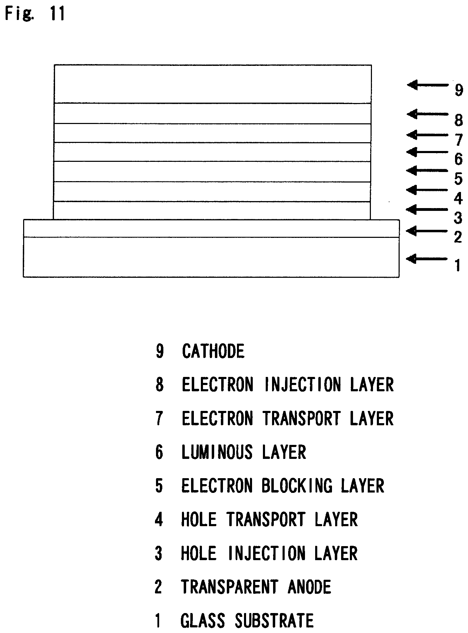

Fig. 11, for example, shows the layer constitution of an organic EL device in which a transparent anode 2, a hole injection layer 3, a hole transport layer 4, an electron blocking layer 5, a luminous layer 6, an electron transport layer 7, an electron injection layer 8, and a cathode 9 are formed in this sequence on a glass substrate 1. The respective layers constituting the organic EL device of the present invention will be described below.

### (Anode 2)

In the organic EL device of the present invention, an electrode material having a high work function, such as ITO or gold, is used for the anode 2.

### (Hole injection layer 3)

For the hole injection layer 3, a publicly known material, for example, porphyrin compounds typified by copper phthalocyanine; triphenylamine derivatives of starburst type; materials such as various triphenylamine tetramers; acceptor type heterocyclic compounds such as hexacyanoazatriphenylene; and coating type polymeric materials can be used in addition to the arylamine compounds of the present invention.

These materials may be subjected singly to film formation, but a plurality of them may be mixed and subjected to film formation. If the plurality of materials are used in combination, those p-doped with tris(bromophenyl)aminium hexachloroantimonate or radialene derivatives (see WO2014/009310), or polymeric compounds containing the structures of benzidine derivatives in their partial structures such as TPD may also be used.

When thin film formation is performed by a publicly known method such as vapor deposition, a spin coat method or an ink jet method with the use of any of the above materials, the hole injection layer 3 can be obtained. Each of the layers to be described below can similarly be obtained by thin film formation performed using a publicly known method such as vapor deposition, spin coating, or ink jetting.

### (Hole transport layer 4)

For the hole transport layer 4, a publicly known compound having hole transport properties can be used, as well as the arylamine compound of the present invention. The publicly known compound having hole transport properties can be exemplified by the following:
benzidine derivatives, for example,
   N,N'-diphenyl-N,N'-di(m-tolyl)benzidine (TPD),
   N,N'-diphenyl-N,N'-di(α-naphthyl)benzidine (NPD), and
   N,N,N' ,N'-tetrabiphenylylbenzidine;
1,1-bis[4-(di-4-tolylamino)phenyl]cyclohexane (TAPC); and
various triphenylamine trimers or tetramers.

These materials may be subjected singly to film formation, but a plurality of them may be mixed and subjected to film formation. The hole transport layer 4 may have a single-layer structure or a structure constituted by a plurality of layers.

For the hole transport layer 4, it is also allowable to use the material usually used for the layer and p-doped with tris(bromophenyl)aminium hexachloroantimonate or a radialene derivative (see, for example, WO2014/009310), or to use a polymeric compound containing the structure of a benzidine derivative in its partial structure such as TPD.

In forming the hole injection layer concurrently serving as the hole transport layer, a coating type polymeric material such as poly(3,4-ethylenedioxythiophene) (PEDOT) /poly(styrene sulfonate)(PSS) can be used.

### (Electron blocking layer 5)

For the electron blocking layer 5, publicly known compounds having electron blocking action can be used in addition to the arylamine compound of the present invention. As the publicly known compounds having electron blocking action, the following can be exemplified:
Carbazole derivatives, for example,
   4,4',4"-tri(N-carbazolyl)triphenylamine (TCTA);
   9,9-bis[4-(carbazol-9-yl)phenyl]fluorene;
   1,3-bis(carbazol-9-yl)benzene (mCP); and
   2,2-bis(4-carbazol-9-ylphenyl)adamantane (Ad-Cz); and
Compounds having a triphenylsilyl group and a triarylamine structure, for example,
9-[4-(carbazol-9-yl)phenyl]-9-[4-(triphenylsilyl)phenyl]-9H-fluorene.

These materials may be subjected singly to film formation, but a plurality of them may be mixed and subjected to film formation. The electron blocking layer 5 may have a single-layer structure, or a structure constituted by a plurality of layers.

### (Luminous layer 6)

For the luminous layer 6, publicly known luminous materials can be used. Examples of the publicly known luminous materials include various metal complexes, such as metal complexes of quinolinol derivatives including Alq₃; anthracene derivatives; bisstyrylbenzene derivatives; pyrene derivatives; oxazole derivatives; and polyparaphenylenevinylene derivatives.

The luminous layer 6 may be comprised with a host material and a dopant material. Examples of the host material include, in addition to the arylamine compounds of the present invention and the above luminous materials, heterocyclic compounds having an indole ring as a partial structure of a condensed ring; heterocyclic compounds having a carbazole ring as a partial structure of a condensed ring; carbazole derivatives; thiazole derivatives; benzimidazole derivatives; polydialkylfluorene derivatives; and anthracene derivatives.

As the dopant material, there can be used amine derivatives having a fluorene ring as a partial structure of a condensed ring; quinacridone, coumarin, rubrene, perylene, pyrene and derivatives thereof; benzopyran derivatives; indenophenanthrene derivatives; rhodamine derivatives; aminostyryl derivatives; and the like.

These materials may be subjected singly to film formation, but a plurality of them may be mixed and subjected to film formation. The luminous layer 6 may have a single-layer structure, or a structure constituted by a plurality of layers.

Furthermore, a phosphorescent luminous body can be used as the luminous material. As the phosphorescent luminous body, a phosphorescent luminous body in the form of a metal complex containing iridium, platinum or the like can be used. Concretely, a green phosphorescent luminous body such as Ir(ppy)₃; a blue phosphorescent luminous body such as FIrpic or FIr6; a red phosphorescent luminous body such as Btp₂Ir(acac); or the like can be used.

As the host material in this case, the following hole injecting/transporting host material, for example, can be used:
A carbazole derivative, for example, 4,4'-di(N-carbazolyl)biphenyl (CBP), TCTA, or mCP; or
Arylamine compound of the present invention.
The following electron transporting host material is also usable:
p-bis(triphenylsilyl)benzene (UGH2); or
2,2',2"-(1,3,5-phenylene)-tris(1-phenyl-1H-benzimidaz ole) (TPBI).
By using such a host material, a high performance organic EL device can be prepared.

To avoid concentration quenching, the host material is desirably doped with the phosphorescent luminous material by the vacuum coevaporation in a range of 1 to 30% by weight based on the entire luminous layer.

Moreover, a material which emits delayed fluorescence, such as a CDCB derivative, for example, PIC-TRZ, CC2TA, PXZ-TRZ, or 4CzIPN, can be used as the luminous material.

### (Hole blocking layer)

A hole blocking layer (not shown) can be provided between the luminous layer 6 and the electron transport layer 7. The hole blocking layer can use publicly known compounds having hole blocking action. The publicly known compounds having hole blocking action are as follows:
Phenanthroline derivatives, e.g., bathocuproine (BCP);
Metal complexes of quinolinol derivatives, e.g., aluminum(III) bis(2-methyl-8-quinolinolato)-4-phenylphenolate (BAlq);
Various rare earth complexes;
Triazole derivatives;
Triazine derivatives; and
Oxadiazole derivatives.

These materials may also concurrently serve as the materials for the electron transport layer. These materials may be subjected singly to film formation, but a plurality of them may be mixed and subjected to film formation. The hole blocking layer may have a single-layer structure, or a structure constituted by a plurality of layers.

### (Electron transport layer 7)

The electron transport layer 7 can use the following publicly known compounds:
Metal complexes of quinolinol derivatives including Alq₃ and BAIq;
Various metal complexes;
Triazole derivatives;
Triazine derivatives;
Oxadiazole derivatives;
Pyridine derivatives;
Pyrimidine derivatives;
Benzimidazole derivatives;
Thiadiazole derivatives;
Anthracene derivatives;
Carbodiimide derivatives;
Quinoxaline derivatives;
Pyridoindole derivatives;
Phenanthroline derivatives; and
Silole derivatives.

These materials may be subjected singly to film formation, but a plurality of them may be mixed and subjected to film formation. The electron transport layer 7 may have a single-layer structure, or a structure constituted by a plurality of layers.

### (Electron injection layer 8)

The electron injection layer 8 can use an alkali metal salt such as lithium fluoride or cesium fluoride; an alkaline earth metal salt such as magnesium fluoride; or a metal oxide such as aluminum oxide. However, the electron injection layer can be omitted in the suitable selection of the electron transport layer and the cathode.

### (Cathode 9)

In the cathode 9, a metal with a low work function such as aluminum, or an alloy having a lower work function, such as a magnesium-silver alloy, a magnesium-indium alloy, or an aluminum-magnesium alloy, is used as an electrode material.

### Examples

The embodiments of the present invention will now be described more specifically by the following Examples, but the present invention is in no way limited to these Examples.

### <Example 1: Compound 2>

### Synthesis of N,N-bis(biphenyl-4-yl)-N-[4-{(2,4,6-triphenyl)phenyl}phenyl ] amine:

### (Procedure 1)

A nitrogen-purged reaction vessel was charged with

| | |
|---|---|
| 1, 3, 5-triphenylbenzene | 50.7 g, |
| and | |
| chloroform | 500 ml. |

Then,

| | |
|---|---|
| bromine | 29.1 g |

was added, and the mixture was stirred for 16 hours at room temperature to prepare a reaction liquid. To the reaction liquid, a saturated aqueous solution of sodium sulfite was added, followed by stirring the mixture. Then, a liquid separating operation was performed, and an organic layer was collected. The organic layer was dehydrated over magnesium sulfate, and then concentrated under reduced pressure to obtain a crude product. Hexane was added to the crude product, and the mixture was dispersed and washed. As a result, 55.0 g (yield 86%) of 2-bromo-1, 3, 5-triphenylbenzene was obtained as a white powder.

### (Procedure 2)

| | |
|---|---|
| A nitrogen-purged reaction vessel was charged with the resulting 2-bromo-1,3,5-triphenylbenzene | 5.0 g, |
| 4-{N,N-bis(biphenyl-4-yl)amino}phenylboronic acid | 6.9 g, |
| tripotassium phosphate | 8.3 g, |
| 1,4-dioxane | 90 ml, |
| and | |
| water | 10 ml. |

A nitrogen gas was passed through the vessel for 30 minutes to obtain a mixture. To this mixture,

| | |
|---|---|
| palladium(II) acetate | 0.087 g, |
| and | |
| tricyclohexylphosphine | 0.25 g |

were added, followed by heating. The mixture was stirred for 6 hours at 85°C to obtain a reaction liquid. To the reaction liquid, 50 ml of water was added, and precipitated solids were collected by filtration to obtain a crude product. Toluene was added to the resulting crude product, followed by heating the mixture until it dissolved. Silica gel was added, and the mixture was stirred, whereafter hot filtration was performed. The filtrate was cooled to room temperature, and precipitated solids were collected by filtration. The filter cake was subjected to recrystallization using toluene. As a result, 7.7 g (yield 84%) of Compound 2 was obtained as a white powder.

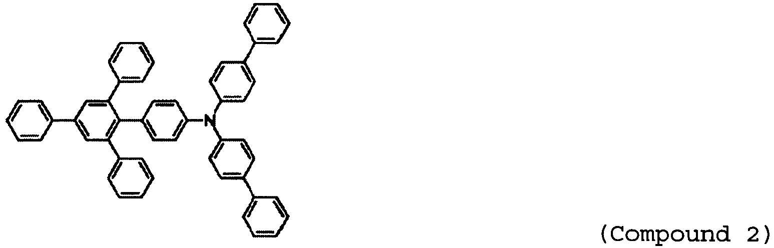

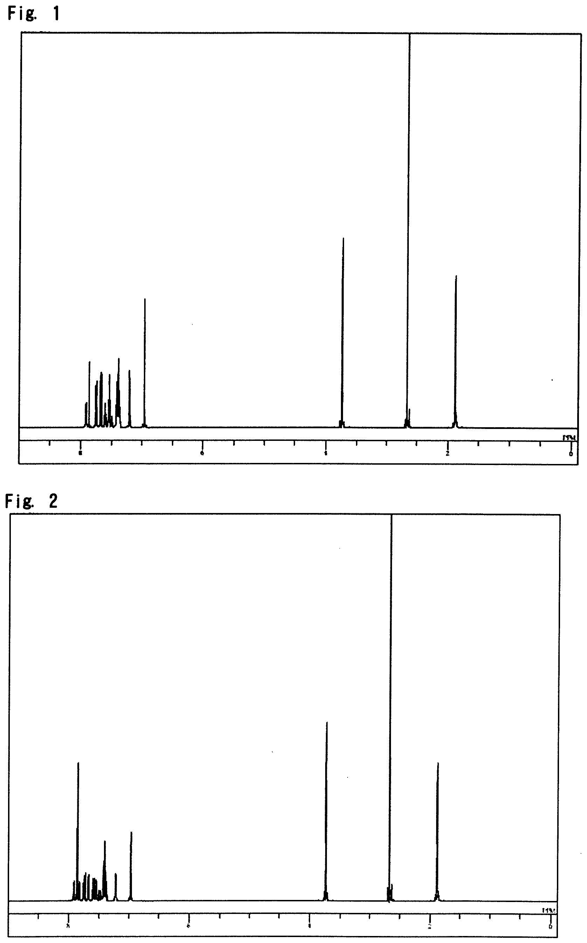

The structure of the resulting white powder was identified using NMR. The results of the ¹H-NMR measurement are shown in Fig. 1. In the ¹H-NMR (THF-d₈), the following signals of 39 hydrogens were detected.

| | |
|---|---|
| δ (ppm) | = 7.92 (2H) |
| | 7.87 (2H) |
| | 7.75 (4H) |
| | 7.67 (4H) |
| | 7.60 (2H) |
| | 7.54 (4H) |
| | 7.49 (1H) |
| | 7.40 (12H) |
| | 7.21 (4H) |
| | 6.96 (4H) |

### <Example 2: Compound 10>

### Synthesis of N-(biphenyl-4-yl)-N-(1,1' :4' ,1"-terphenyl-4-yl)-N-[4-{(2,4, 6-triphenyl)phenyl}phenyl]amine:

A nitrogen-purged reaction vessel was charged with

| | |
|---|---|
| N-(4-bromophenyl)-4-biphenylamine | 38.0 g, |
| 4-biphenylboronic acid | 25.5 g, |
| potassium carbonate | 32.4 g, |
| toluene | 3000 ml, |
| ethanol | 76 ml, and |
| water | 113 ml. |

A nitrogen gas was passed through the vessel for 30 minutes to obtain a mixture. To this mixture,

| | |
|---|---|
| tetrakis(triphenylphosphine)palladium | 2.7 g, |

was added, followed by heating, and the mixture was stirred for 5 hours at 73°C to obtain a reaction liquid. To the reaction liquid, 100 ml of water was added, and precipitated solids were collected by filtration. To the resulting solids,
o-dichlorobenzene was added, followed by heating the mixture until it dissolved. Further, silica gel was added, and the mixture was stirred, whereafter hot filtration was performed. The filtrate was concentrated under reduced pressure, and precipitated solids were collected by filtration. As a result, 20.1 g (yield 43%) of N-(biphenyl-4-yl)-N-(1,1':4',1"-terphenyl-4-yl)amine was obtained as a yellow powder.

A nitrogen-purged reaction vessel was charged with the resulting

| | |
|---|---|
| N-(biphenyl-4-yl)-N-(1,1':4',1"-terphenyl-4-yl)amine | 20.0 g, |
| iodobenzene | 15.4 g, |
| copper powder | 0.3 g, |
| potassium carbonate | 13.9 g, |
| 3,5-di-tert-butylsalicylic acid | 1.2 g, |
| sodium bisulfite | 1.5 g, and |
| dodecylbenzne | 20 ml. |

The mixture was heated, and stirred for 16 hours at 180°C to obtain a reaction liquid. The reaction liquid was cooled to 100°C, then toluene was added, and precipitated solids were collected by filtration. The collected solids were washed with water, and then washed with methanol. The washed solids were dissolved in o-dichlorobenzene, and the solution was subjected to adsorption purification using silica gel. As a result, 17.1 g (yield 72%) of
N-(biphenyl-4-yl)-N-phenyl-N-(1,1':4' ,1"-terphenyl-4-yl) ami ne was obtained as a white powder.

A nitrogen-purged reaction vessel was charged with

| | |
|---|---|
| the resulting N-(biphenyl-4-yl)-N-phenyl-N-(1,1':4',1"-terphenyl-4-yl)amine | 17.0 g, and |
| dimethylformamide | 340 ml. |

Then,

| | |
|---|---|
| N-bromosuccinimide | 7.0 g |

was added, followed by stirring for 13 hours at room temperature to obtain a mixture. Methanol was added to the mixture, and precipitated solids were collected by filtration. As a result, 17.2 g (yield 87%) of N-(biphenyl-4-yl)-N-(4-bromophenyl)-N-(1,1':4',1"-terphenyl -4-yl)amine was obtained as a white powder.

A nitrogen-purged reaction vessel was charged with the resulting

| | |
|---|---|
| N-(biphenyl-4-yl)-N-(4-bromophenyl)-N-(1,1':4',1"-terphenyl-4-yl)amine | 5.0 g, |
| bis(pinacolato)diboron | 2.8 g, |
| potassium acetate | 2.2 g, and |
| 1,4-dioxane | 100 ml. |

A nitrogen gas was passed through the vessel for 30 minutes to obtain a mixture. 0.2 g of a dichloromethane adduct of {1,1'-bis(diphenylphosphino)ferrocene}palladium(II) dichloride was added to the mixture, followed by heating. The heated mixture was stirred for 5 hours at 97°C to obtain a reaction liquid. The resulting reaction liquid was cooled to room temperature, and a liquid separating operation was performed with the addition of water and toluene to collect an organic layer. The organic layer was dehydrated over anhydrous magnesium sulfate, and then concentrated under reduced pressure to obtain a crude product. The crude product was dissolved in toluene, and the solution was subjected to adsorption purification using silica gel. After filtration, the filtrate was concentrated under reduced pressure, and precipitated solids were collected by filtration. As a result, 4.4 g (yield 81%) of N-(biphenyl-4-yl)-N-{4-(4,4,5,5-tetramethyl-[1,3,2]dioxabor an-2-yl)phenyl}-N-(1,1':4',1"-terphenyl-4-yl)amine was obtained as a gray powder.

In Procedure 2 of Example 1,
N-(biphenyl-4-yl)-N-{4-(4,4,5,5-tetramethyl-[1,3,2]dioxaboran-2-yl)phenyl}-N-(1,1':4',1"-terphenyl-4-yl) amine was used instead of
4-{N,N-bis(biphenyl-4-yl)amino}phenylboronic acid, and reactions were performed under the same conditions. As a result, 3.8 g (yield 75%) of Compound 10 was obtained as a white powder.

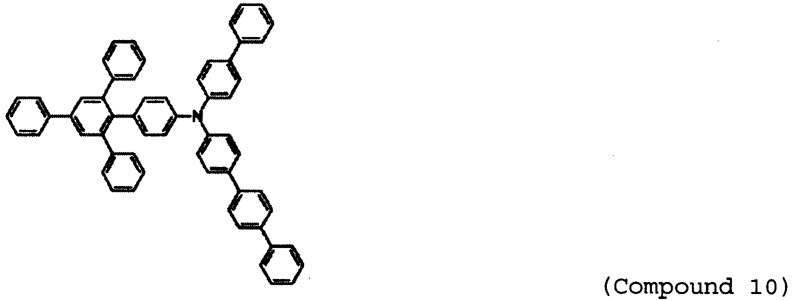

The structure of the resulting white powder was identified using NMR. The results of the ¹H-NMR measurement are shown in Fig. 2. In the ¹H-NMR (THF-d₈), the following signals of 43 hydrogens were detected.

| | |
|---|---|
| δ (ppm) | = 7.92 (2H) |
| | 7.87 (2H) |
| | 7.85 (4H) |
| | 7.83 (2H) |
| | 7.74 (4H) |
| | 7.68 (2H) |
| | 7.57 (6H) |
| | 7.49 (2H) |
| | 7.40 (11H) |
| | 7.22 (4H) |
| | 6.97 (4H) |

### <Example 3: Compound 41>

### Synthesis of

### N-(biphenyl-4-yl)-N-(9,9-dimethyl-9H-fluoren-2-yl)-N-[4-{(2 ,4,6-triphenyl)phenyl}phenyl]amine:

| | |
|---|---|
| N-(biphenyl-4-yl)-N-(4-bromophenyl)-N-(9,9-dimethyl-9H-fluoren-2-yl)amine | 71.9 g, and |
| tetrahydrofuran | 360 ml, |

were put into a nitrogen-purged reaction vessel and was cooled to -78°C to obtain a mixture. To this mixture, 100 ml of a hexane solution (1.6M) of n-butyl lithium was slowly added dropwise, followed by stirring the mixture for 1 hour at the same temperature. Then, 19 ml of trimethyl borate was slowly added dropwise, and the mixture was stirred for 1 hour at the same temperature. Then, the mixture was heated to room temperature, and then further stirred for 1 hour. Then, a 1N aqueous solution of hydrochloric acid was added, and the mixture was stirred for 1 hour. By performing a liquid separating operation, an organic layer was collected. Then, the organic layer was dehydrated over anhydrous magnesium sulfate, and then concentrated under reduced pressure to obtain a crude product. The resulting crude product was subjected to crystallization purification using an ethyl acetate/n-hexane mixed solution. As a result, 44.6 g (yield 67%) of 4-{N-(biphenyl-4-yl)-N-(9,9-dimethyl-9H-fluoren-2-yl)amino} phenylboronic acid was obtained as a gray powder.

In Procedure 2 of Example 1,
4-{N-(biphenyl-4-yl)-N-(9,9-dimethyl-9H-fluoren-2-yl) amino}phenylboronic acid
was used instead of
4-{N,N-bis(biphenyl-4-yl)amino}phenylboronic acid, and reactions were performed under the same conditions. As a result, 4.9 g (yield 85%) of Compound 41 was obtained as a white powder.

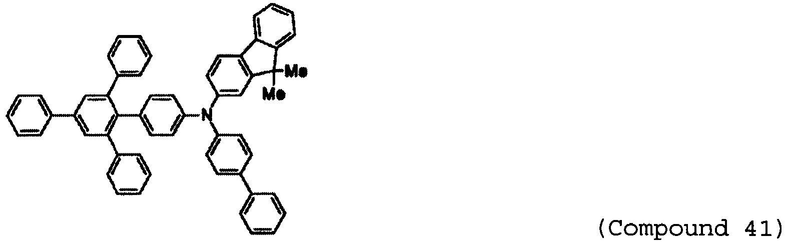

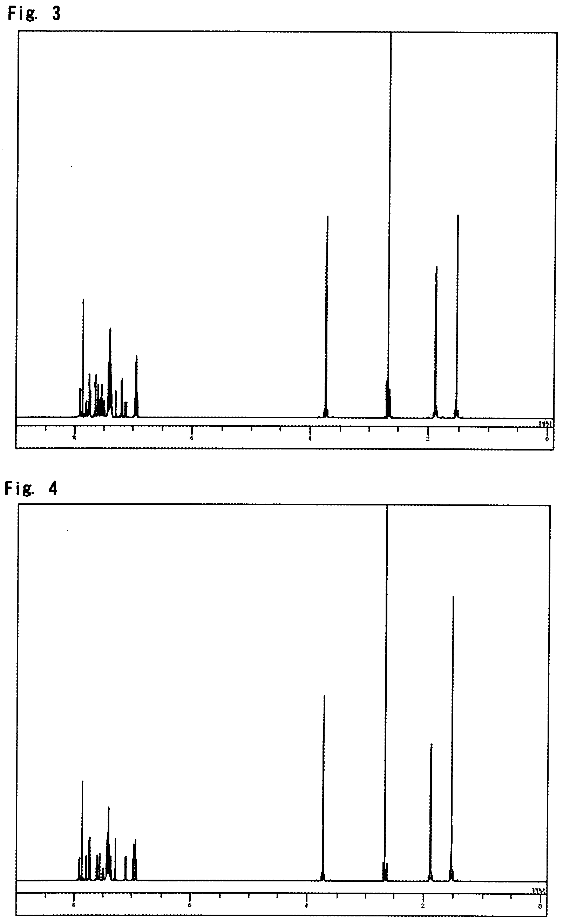

The structure of the resulting white powder was identified using NMR. The results of the ¹H-NMR measurement are shown in Fig. 3. In the ¹H-NMR (THF-d₈), the following signals of 43 hydrogens were detected.

| | |
|---|---|
| δ (ppm) | = 7.92 (2H) |
| | 7.87 (2H) |
| | 7.81 (1H) |
| | 7.76 (3H) |
| | 7.65 (2H) |
| | 7.60 (2H) |
| | 7.57-7.52 (3H) |
| | 7.50 (1H) |
| | 7.40 (13H) |
| | 7.29 (1H) |
| | 7.19 (2H) |
| | 7.13 (1H) |
| | 6.95 (4H) |
| | 1.55 (6H) |

### <Example 4: Compound 42>

### Synthesis of N,N-bis(9,9-dimethyl-9H-fluoren-2-yl)-N-[4-{ (2,4,6-tripheny l)phenyl}phenyl]amine:

In Example 1,
N,N-bis(9,9-dimethyl-9H-fluoren-2-yl)-N-{4-(4,4,5,5-t etramethyl-[1,3,2]dioxaboran-2-yl)phenyl}amine was used instead of
4-{N,N-bis(biphenyl-4-yl)amino}phenylboronic acid, and reactions were performed under the same conditions. As a result, 11.0 g (yield 91%) of Compound 42 was obtained as a white powder.

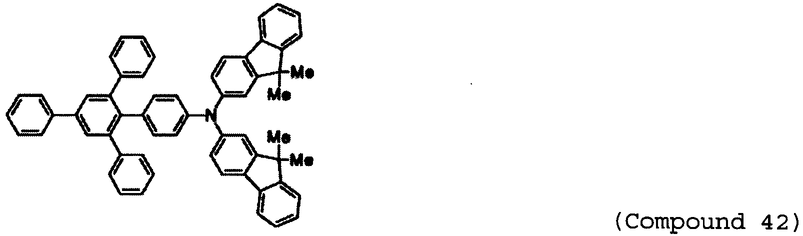

The structure of the resulting white powder was identified using NMR. The results of the ¹H-NMR measurement are shown in Fig. 4. In the ¹H-NMR (THF-d₈), the following signals of 47 hydrogens were detected.

| | |
|---|---|
| δ (ppm) | = 7.92 (2H) |
| | 7.87 (2H) |
| | 7.80 (2H) |
| | 7.74 (2H) |
| | 7.60 (2H) |
| | 7.56 (2H) |
| | 7.50 (1H) |
| | 7.40 (14H) |
| | 7.29 (2H) |
| | 7.10 (2H) |
| | 6.97 (2H) |
| | 6.93 (2H) |
| | 1.55 (12H) |

### <Example 5: Compound 57>

### Synthesis of N-(biphenyl-4-yl)-N-{p-(9,9-dimethyl-9H-fluoren-2-yl)phenyl }-N-[4-{(2,4,6-triphenyl)phenyl}phenyl]amine:

A nitrogen-purged reaction vessel was charged with

| | |
|---|---|
| N-(4-bromophenyl)-N-(biphenyl)amine | 20.0 g, |
| 2-(9,9-dimethyl-9H-fluoren-2-yl)-4,4,5,5-tetramethyl-1,3,2-dioxaborane | 21.7 g, |
| tripotassium phosphate | 26.2 g, |
| 1,4-dioxane | 200 ml, and |
| water | 70 ml, |

and a nitrogen gas was passed through the vessel for 40 minutes to obtain a mixture. To the mixture, 1.4 g of tetrakis(triphenylphosphine)palladium was added, followed by heating. The mixture was stirred for 4 hours at 85°C to obtain a reaction liquid. The reaction liquid was cooled to room temperature, whereafter a liquid separating operation was performed with the addition of toluene to collect an organic layer. The organic layer was dried over magnesium sulfate, and then concentrated under reduced pressure to obtain a crude product. Hexane was added to the resulting crude product, and the mixture was subjected to crystallization purification, whereupon precipitated solids were collected by filtration. As a result, 20.7 g (yield 77%) of N-(biphenyl-4-yl)-N-[p-(9,9-dimethyl-9H-fluoren-2-yl)phenyl ]amine was obtained as white solids.

A nitrogen-purged reaction vessel was charged with

| | |
|---|---|
| the resulting N- (biphenyl-4-yl) -N- [p- (9, 9-dimethyl-9H-fluoren-2-yl)phenyl]amine | 20.0 g, |
| 1-bromo-4-iodebenzene | 15.5 g, |
| sodium tert-butoxide | 6.6 g, and |
| xylene | 400 ml, |

and a nitrogen gas was passed through the vessel for 1 hour to obtain a mixture. To the mixture,

| | |
|---|---|
| palladium acetate | 0.2 g, and |
| xantphos | 1.1 g, |

were added, followed by heating. The mixture was stirred for 2 hours at 105°C to obtain a reaction liquid. The reaction liquid was cooled to room temperature, then water was added, and the mixture was filtered to collect an organic layer. The organic layer was dried over magnesium sulfate, and then concentrated under reduced pressure to obtain a concentrate. The concentrate was subjected to crystallization purification using methanol, whereupon precipitated solids were collected by filtration. As a result, 19.0 g (yield 70%) of N-(biphenyl-4-yl)-N-(4-bromophenyl)-N-[p-(9,9-dimethyl-9H-f luoren-2-yl)phenyl]amine was obtained as white solids.

| | |
|---|---|
| The resulting N-(biphenyl-4-yl)-N-(4-bromophenyl)-N-[p-(9,9-dimethyl-9H-fluoren-2-yl)phenyl]amine | 18.0 g, |
| and | |
| tetrahydrofuran | 20 ml, |

were put into a nitrogen-purged reaction vessel and cooled to -68°C to obtain a mixture. To this mixture, 22 ml of a hexane solution (1.6M) of n-butyl lithium was added dropwise over 45 minutes, followed by stirring the mixture for 45 minutes . Then, 4 ml of trimethyl borate was added dropwise over 15 minutes, and then the mixture was stirred for 1 hour at -68°C. The mixture was further stirred for 2 hours at room temperature, 60 ml of 1N hydrochloric acid was added, and the mixture was stirred for 2 hours . An organic layer was dehydrated over magnesium sulfate, and then concentrated under reduced pressure. The resulting concentrate was subjected to crystallization purification using hexane, and precipitated solids were collected by filtration. As a result, 12.5 g (yield 74%) of 4-[N-(biphenyl-4-yl)-N-{p-(9,9-dimethyl-9H-fluoren-2-yl)phe nyl}amino}phenylboronic acid was obtained as a white powder.

In Procedure 2 of Example 1,
4-[N-(biphenyl-4-yl)-N-{p-(9,9-dimethyl-9H-fluoren-2-yl)phenyl}amino}phenylboronic acid was used instead of
4-{N,N-bis(biphenyl-4-yl)amino}phenylboronic acid, and reactions were performed under the same conditions. As a result, 5.5 g (yield 43%) of Compound 57 was obtained as white solids.

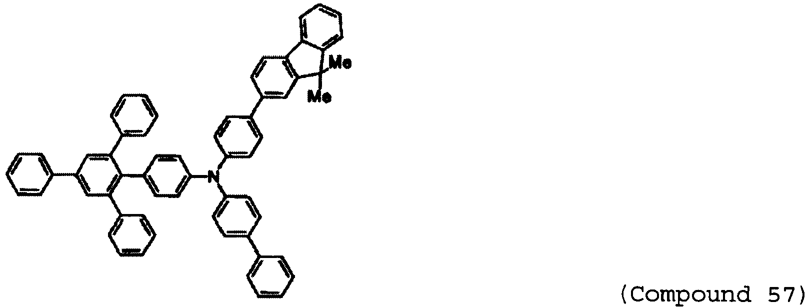

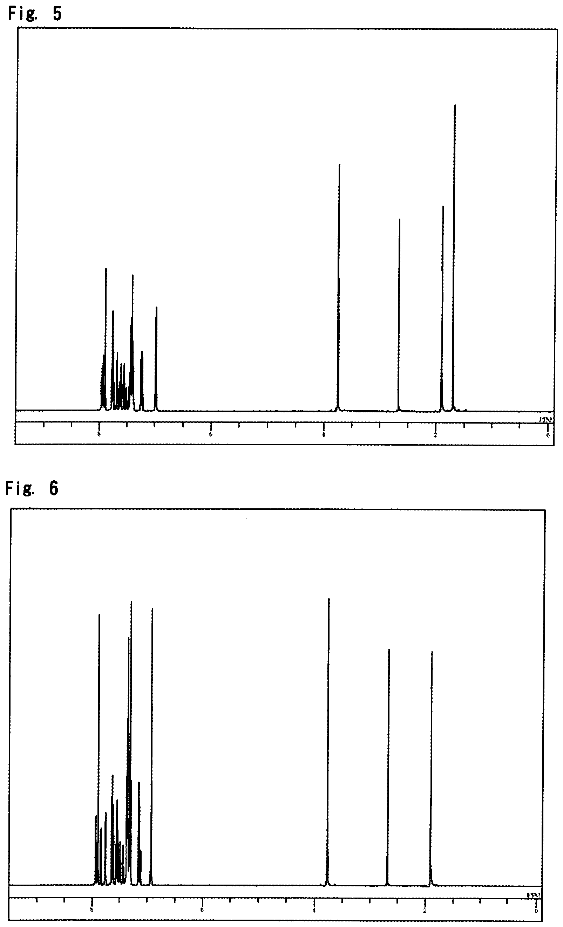

The structure of the resulting white solids was identified using NMR. The results of the ¹H-NMR measurement are shown in Fig. 5. In the ¹H-NMR (THF-d₈), the following signals of 47 hydrogens were detected.

| | |
|---|---|
| δ (ppm) = | 7.97-7.92 (4H) |
| | 7.90 (3H) |
| | 7.77 (5H) |
| | 7.70 (2H) |
| | 7.63 (3H) |
| | 7.57 (2H) |
| | 7.52 (1H) |
| | 7.44 (13H) |
| | 7.25 (4H) |
| | 6.99 (4H) |
| | 1.71 (6H) |

### <Example 6: Compound 62>

### Synthesis of N-(biphenyl-4-yl)-N-(9,9-diphenyl-9H-fluoren-2-yl)-N-[4-{(2 ,4,6-triphenyl)phenyl}phenyl]amine:

A nitrogen-purged reaction vessel was charged with

| | |
|---|---|
| 2-amino-9,9-biphenyl-9H-fluorene | 10.0 g, |
| 4-bromobiphenyl | 7.3 g, |
| sodium tert-butoxide | 4.3 g, |
| and | |
| toluene | 100 ml, |

and a nitrogen gas was passed through the vessel for 40 minutes to obtain a mixture. To the mixture,

| | |
|---|---|
| palladium acetate | 0.1 g, |
| and | |
| a toluene solution (50 wt.%) of tri-tert-butylphosphine | 0.7 g |

were added, followed by heating. The mixture was stirred for 5 hours at 80°C to obtain a reaction liquid. The reaction liquid was cooled to room temperature, and then concentrated under reduced pressure. Toluene was added to the concentrate, and the mixture was dissolved by heating. Silica gel was added, and the mixture was stirred and hot-filtered. The filtrate was concentrated under reduced pressure, and precipitated solids were collected by filtration. As a result, 12.0 g (yield 82%) of N-(biphenyl-4-yl)-N-(9,9-diphenyl-9H-fluoren-2-yl)amine was obtained as white solids.

A nitrogen-purged reaction vessel was charged with

| | |
|---|---|
| the resulting N-(biphenyl-4-yl)-N-(9,9-diphenyl-9H-fluoren-2-yl)amine | 11.9 g, |
| 1-bromo-4-iodebenzene | 8.3 g, |
| sodium tert-butoxide | 3.5 g, |
| and | |
| xylene | 240 ml, |

and a nitrogen gas was passed through the vessel for 1 hour to obtain a mixture. To the mixture,

| | |
|---|---|
| palladium acetate | 0.1 g, |
| and | |
| xantphos | 0.6 g, |

were added, followed by heating. The mixture was stirred for 4 hours at 120°C to obtain a reaction liquid. The reaction liquid was cooled to room temperature, then water was added, and the mixture was filtered to collect an organic layer. The organic layer was dried over magnesium sulfate, and then concentrated under reduced pressure. The concentrate was subjected to adsorption purification using silica gel. As a result, 13.2 g (yield 84%) of N-(biphenyl-4-yl)-N-(4-bromophenyl)-N-(9,9-diphenyl-9H-fluo ren-2-yl)amine was obtained as white solids.

| | |
|---|---|
| The resulting N-(biphenyl-4-yl)-N-(4-bromophenyl)-N-(9,9-diphenyl-9H-fluoren-2-yl)amine and | 12.9 g, |
| tetrahydrofuran | 100 ml, |

were put into a nitrogen-purged reaction vessel and cooled to -68°C to obtain a mixture. To this mixture, 15 ml of a hexane solution (1.6M) of n-butyl lithium was added dropwise over 20 minutes, followed by stirring the mixture for 40 minutes. Then, 3 ml of trimethyl borate was added dropwise over 15 minutes, and the mixture was stirred for 1 hour at -68°C. The mixture was further stirred for 2 hours at room temperature, 60 ml of 1N hydrochloric acid was added, followed by stirring the mixture for 2 hours. An organic layer was dehydrated over magnesium sulfate, and then concentrated under reduced pressure. The resulting concentrate was subjected to crystallization purification using hexane, and precipitated solids were collected by filtration. As a result, 7.3 g (yield 60%) of 4-{N-(biphenyl-4-yl)-N-(9,9-diphenyl-9H-fluoren-2-yl)amino} phenylboronic acid was obtained as greenish white solids.

In Procedure 2 of Example 1,
4-{N-(biphenyl-4-yl)-N-(9,9-diphenyl-9H-fluoren-2-yl) amino}phenylboronic acid
was used instead of
4-{N,N-bis(biphenyl-4-yl)amino}phenylboronic acid, and reactions were performed under the same conditions. As a result, 7.5 g (yield 81%) of Compound 62 was obtained as yellowish white solids.

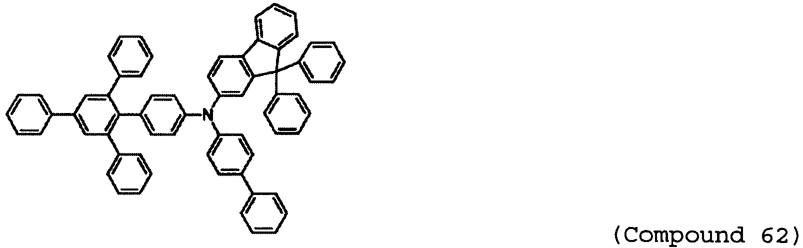

The structure of the resulting yellowish white solids was identified using NMR. The results of the ¹H-NMR measurement are shown in Fig. 6. In the ¹H-NMR (THF-d₈), the following signals of 47 hydrogens were detected.

| | |
|---|---|
| δ (ppm) = | 7.93 (2H) |
| | 7.90 (1H) |
| | 7.88 (2H) |
| | 7.83 (1H) |
| | 7.75 (2H) |
| | 7.64-7.61 (4H) |
| | 7.55 (3H) |
| | 7.53-7.48 (2H) |
| | 7.43 (1H) |
| | 7.36 (11H) |
| | 7.31 (11H) |
| | 7.17 (2H) |
| | 7.14 (1H) |
| | 6.94 (4H) |

### <Example 7: Compound 91>

### Synthesis of N-{p-(phenanthrenyl-9-yl)phenyl}-N-(1,1':4',1"-terphenyl-4-yl)-N-[4-{(2,4,6-triphenyl)phenyl}phenyl]amine:

A nitrogen-purged reaction vessel was charged with

| | |
|---|---|
| 4-bromodiphenylamine | 100.0 g, |
| bis(pinacolato)diboron | 123.0 g, |
| potassium acetate | 98.9 g, |
| and | |
| 1,4-dioxane | 1000 ml. |

A nitrogen gas was passed through the vessel for 30 minutes to obtain a mixture. To the mixture, 6.6 g of {1,1'-bis(diphenylphosphino)ferrocene}palladium was added, followed by heating. The heated mixture was stirred for 6 hours at 100°C to obtain a reaction liquid. The resulting reaction liquid was cooled to room temperature, and then a liquid separating operation was performed with the addition of toluene and water to collect an organic layer. The organic layer was dehydrated over magnesium sulfate, and then concentrated under reduced pressure . Hexane was added to the concentrate, and the mixture was cooled in a freezer, whereafter precipitated solids were collected by filtration. As a result, 56.1 g (yield 47%) of N-phenyl{p-(4,4,5,5-tetramethyl-1,3,2-dioxaboran-2-yl)pheny 1}amine was obtained as white solids.

A nitrogen-purged reaction vessel was charged with

| | |
|---|---|
| the resulting N-phenyl{p-(4,4,5,5-tetramethyl-1,3,2-dioxaboran-2-yl)phenyl}amine | 54.8g, |
| 2-bromo-1,3,5-triphenylbenzene | 65.0 g, |
| tripotassium phosphate | 71.7 g, |
| 1,4-dioxane | 650 ml, |
| and | |
| water | 180 ml. |

A nitrogen gas was passed through the vessel for 45 minutes to obtain a mixture. To this mixture, 3.9 g of tetrakis(triphenylphosphine)palladium was added, followed by heating, and the mixture was stirred for 11 hours at 90°C to obtain a reaction liquid. After the reaction liquid was cooled to room temperature, water was added, and a liquid separating operation was performed to collect an organic layer. The organic layer was dehydrated over magnesium sulfate, and then concentrated under reduced pressure to obtain a crude product. Methanol was added to the crude product, and the mixture was dispersed and washed. As a result, 71.6 g (yield 90%) of N-phenyl-N-[4-{(2,4,6-triphenyl)phenyl}phenyl]amine was obtained as white solids.

A nitrogen-purged reaction vessel was charged with

| | |
|---|---|
| the resulting N-phenyl-N-[4-{(2,4,6-triphenyl)phenyl}phenyl]amine | 30.0 g, |
| 4-bromo-1,1':4',1"-terphenyl | 20.6 g, |
| sodium tert-butoxide | 9.1 g, |
| and | |
| toluene | 300 ml, |

and a nitrogen gas was passed through the vessel for 35 minutes to obtain a mixture. To the mixture,

| | |
|---|---|
| palladium acetate | 0.3 g, |
| and | |
| a toluene solution (50 wt.%) of tri-tert-butyl phosphine | 1.5 g |

were added, followed by heating. The mixture was stirred for 3 hours at 80°C to obtain a reaction liquid. The reaction liquid was cooled to room temperature, and then concentrated under reduced pressure. The concentrate was subjected to crystallization purification using methanol, and precipitated solids were collected by filtration. As a result, 37.9 g (yield 85%) of N-phenyl-N-(1,1':4',1"-terphenyl-4-yl)-N-[4-f(2,4,6-triphen yl)phenyl}phenyl] amine was obtained as yellowish green solids.

| | |
|---|---|
| The resulting N-phenyl-N-(1,1':4',1"-terphenyl-4-yl)- N-[4-{(2,4,6-triphenyl)phenyl}phenyl]amine | 36.9 g, |
| N-bromosuccinimide | 9.1 g, |
| and | |
| chloroform | 370 ml, |

were put into a nitrogen-purged reaction vessel and stirred for 3 hours at room temperature to obtain a mixture. The mixture was concentrated under reduced pressure. Methanol was added, and precipitated solids were collected by filtration. As a result, 31.4 g (yield 76%) of N-(4-bromophenyl)-N- (1,1':4',1"-terphenyl-4-yl)-N-[4-{(2,4, 6-triphenyl)phenyl}phenyl]amine was obtained as yellowish white solids.

A nitrogen-purged reaction vessel was charged with

| | |
|---|---|
| the resulting N-(4-bromophenyl)-N-(1,1' :4' ,1"-terphenyl-4-yl)-N-[4-{(2,4,6-triphenyl)phenyl}phenyl]amine | 6.2 g, |
| 9-phenanthreneboronic acid | 2.1 g, |
| potassium carbonate | 2.2 g, |
| 1,4-dioxane | 60 ml, |
| and | |
| water | 20 ml. |

A nitrogen gas was passed through the vessel for 35 minutes to obtain a mixture. To this mixture, 0.3 g of tetrakis(triphenylphosphine)palladium was added, followed by heating. The mixture was stirred for 14 hours at 85°C to obtain a reaction liquid. After the reaction liquid was cooled to room temperature, 50 ml of water was added, and the mixture was filtered. Precipitated solids were collected by filtration. Toluene was added to the resulting crude product, and the mixture was dissolved by heating. After silica gel was added to the solution, the mixture was stirred, and then hot-filtered. The filtrate was concentrated under reduced pressure, and the concentrate was subjected to adsorption purification using silica gel. As a result, 2.7 g (yield 39%) of Compound 91 was obtained as white solids.

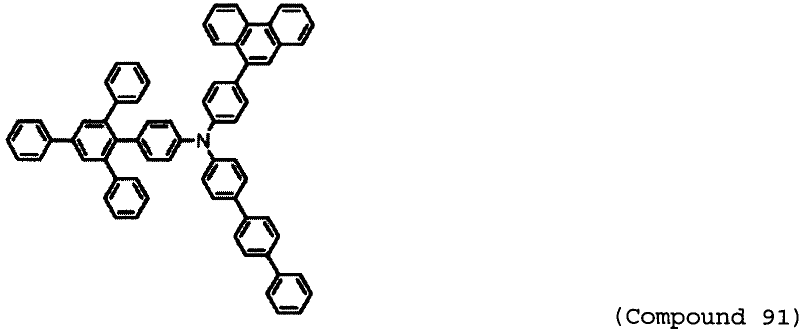

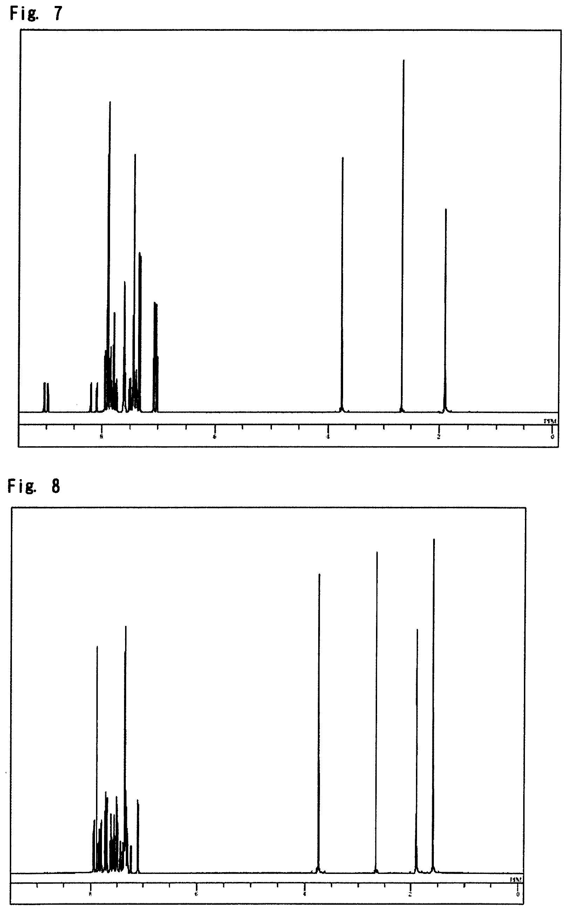

The structure of the resulting white solids was identified using NMR. The results of the ¹H-NMR measurement are shown in Fig. 7. In the ¹H-NMR (THF-d₈), the following signals of 47 hydrogens were detected.

| | | |
|---|---|---|
| δ (ppm) = | 9.04 | (1H) |
| | 8.97 | (1H) |
| | 8.20 | (1H) |
| | 8.10 | (1H) |
| | 7.95 | (2H) |
| | 7.90 | (7H) |
| | 7.85 | (3H) |
| | 7.84-7.82 | (1H) |
| | 7.79 | (3H) |
| | 7.74 | (1H) |
| | 7.61 | (6H) |
| | 7.50 | (2H) |
| | 7.44 | (8H) |
| | 7.39 | (2H) |
| | 7.33 | (4H) |
| | 7.08 | (2H) |
| | 7.03 | (2H) |

### <Example 8: Compound 92>

### Synthesis of N-(biphenyl-4-yl)-N-(9,9-dimethyl-9H-fluoren-2-yl)-N-[4'-{( 2,4,6-triphenyl)-1,1'-biphenyl-4-yl}phenyl]amine:

A nitrogen-purged reaction vessel was charged with

| | |
|---|---|
| 4-{N-(biphenyl-4-yl)-N-(9,9-dimethyl-9H-fluoren-2-yl)amino}phenylboronic acid obtained in Example 3 | 20.0 g, |
| 1-bromo-4-iodobenzene | 12.9 g, |
| potassium carbonate | 11.5 g, |
| toluene | 160 ml, |
| ethanol | 40 ml, and |
| water | 40 ml. |

A nitrogen gas was passed through the vessel for 45 minutes to obtain a mixture. To this mixture, 1.0 g of tetrakis(triphenylphosphine)palladium was added, followed by heating. The mixture was stirred for 8 hours at 74°C to obtain a reaction liquid. After the reaction liquid was cooled to room temperature, water was added, and a liquid separating operation was performed to collect an organic layer. The organic layer was dehydrated over magnesium sulfate, and then concentrated under reduced pressure. Toluene was dissolved in the concentrate, and the solution was added dropwise into methanol. Precipitated solids were collected by filtration. As a result, 19.8 g (yield 80%) of N-(biphenyl-4-yl)-N-(4-bromo-4-biphenyl)-N-(9,9-dimethyl-9H -fluoren-2-yl)amine was obtained as yellowish green solids.

A nitrogen-purged reaction vessel was charged with

| | |
|---|---|
| the resulting N-(biphenyl-4-yl)-N-(4-bromo-4-biphenyl)-N-(9,9-dimethyl-9H-fluoren-2-yl)amine | 10.0 g, |
| bis(pinacolato)diboron | 5.1 g, |
| potassium acetate | 3.3 g, and |
| 1,4-dioxane | 100 ml. |

A nitrogen gas was passed through the vessel for 30 minutes to obtain a mixture. To the mixture, 0.3 g of {1,1'-bis(diphenylphosphino)ferrocene}palladium(II) dichloride was added, followed by heating. The heated mixture was stirred for 19 hours at 80°C to obtain a reaction liquid. The resulting reaction liquid was cooled to room temperature, and then a liquid separating operation was performed with the addition of toluene and water to collect an organic layer. The organic layer was dehydrated over magnesium sulfate, and then concentrated under reduced pressure to obtain a crude product. Methanol was added to the crude product, and the mixture was subjected to crystallization purification. Precipitated solids were collected by filtration. As a result, 7.3 g (yield 68%) of N-(biphenyl-4-yl)-N-(9,9-dimethyl-9H-fluoren-2-yl)-N-{p-(4, 4,5,5-tetramethyl-1,3,2-dioxaboran-2-yl)biphenyl}amine was obtained as white solids.

A nitrogen-purged reaction vessel was charged with

| | |
|---|---|
| the resulting N-(biphenyl-4-yl)-N-(9,9-dimethyl-9H-fluoren-2-yl)-N-{p-(4,4,5,5-tetramethyl-1,3,2-dioxaboran-2-yl)biphenyl}amine | 7.2 g, |
| 2-bromo-1,3,5-triphenylbenzene | 3.6 g, |
| potassium carbonate | 5.2 g, |
| 1,4-dioxane | 80 ml, and |
| water | 20 ml. |

A nitrogen gas was passed through the vessel for 45 minutes to obtain a mixture. To this mixture, 0.3 g of tetrakis(triphenylphosphine)palladium was added, followed by heating. The mixture was stirred for 9 hours at 85°C to obtain a reaction liquid. After the reaction liquid was cooled to room temperature, toluene and water were added, and a liquid separating operation was performed to collect an organic layer. The organic layer was dehydrated over magnesium sulfate, and then concentrated under reduced pressure to obtain a crude product. Toluene and hexane were added to the crude product, and the mixture was subjected to adsorption purification using silica gel. As a result, 6.7 g (yield 88%) of Compound 92 was obtained as yellowish white solids.

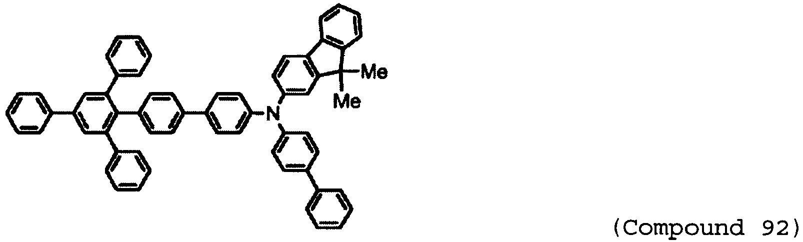

The structure of the resulting yellowish white solids was identified using NMR. The results of the ¹H-NMR measurement are shown in Fig. 8. In the ¹H-NMR (THF-d₈), the following signals of 47 hydrogens were detected.

| | |
|---|---|
| δ (ppm) | = 7.94 (2H) |
| | 7.89 (2H) |
| | 7.84 (2H) |
| | 7.79 (2H) |
| | 7.73 (2H) |
| | 7.69 (2H) |
| | 7.62 (2H) |
| | 7.59-7.55 (3H) |
| | 7.53-7.49 (4H) |
| | 7.46-7.43 (2H) |
| | 7.40 (1H) |
| | 7.33 (14H) |
| | 7.24 (1H) |
| | 7.10 (2H) |
| | 1.59 (6H) |

### <Example 9: Compound 70>

### Synthesis of N-(biphenyl-4-yl)-N-(9-phenyl-9H-carbazol-3-yl)-N-[4-{(2,4, 6-triphenyl)phenyl}phenyl]amine:

### (Procedure 1)

A nitrogen-purged reaction vessel was charged with

| | |
|---|---|
| N-(4-bromobiphenyl)-4-biphenylamine | 50.0 g, |
| bis(pinacolato)diboron | 47.0 g, |
| potassium acetate | 37.8 g, and |
| 1,4-dioxane | 500 ml. |

A nitrogen gas was passed through the vessel for 50 minutes to obtain a mixture. To the mixture, 2.5 g of (1,1'-bis(diphenylphosphino)ferrocene)palladium(II) dichloride was added, followed by heating. The heated mixture was stirred for 5 hours at 180°C to obtain a reaction liquid. After the resulting reaction liquid was cooled to 90°C, a liquid separating operation was performed with the addition of toluene and a saturated saline solution to collect an organic layer. The organic layer was dehydrated over magnesium sulfate, and then concentrated under reduced pressure. Methanol was added to the concentrate, and the mixture was subjected to crystallization purification. Precipitated solids were collected by filtration. As a result, 38.9 g (yield 68%) of N-(biphenyl-4-yl)-N-{4-(4,4,5,5-tetramethyl-1,3,2-dioxabora n-2-yl)phenyl}amine was obtained as yellow solids.

### (Procedure 2)

A nitrogen-purged reaction vessel was charged with

| | |
|---|---|
| the resulting N-(biphenyl-4-yl)-N-{4-(4,4,5,5-tetramethyl-1,3,2-dioxaboran-2-yl)phenyl}amine | 21.2 g, |
| 2-bromo-1,3,5-triphenylbenzene | 20.0 g, |
| tripotassium phosphate | 22.1 g, |
| 1,4-dioxane | 200 ml, and |
| water | 60 ml. |

A nitrogen gas was passed through the vessel for 35 minutes to obtain a mixture. To this mixture, 0.6 g of tetrakis(triphenylphosphine)palladium was added, followed by heating. The heated mixture was stirred for 8 hours at 86°C to obtain a reaction liquid. After the reaction liquid was cooled to room temperature, water was added. Precipitated solids were collected by filtration. Toluene was added to the resulting solids, and the mixture was dissolved by heating. Then, silica gel was added to the solution, the mixture was stirred, and hot filtration was performed. The filtrate was concentrated under reduced pressure, and methanol was added to carry out crystallization purification. Precipitated solids were collected by filtration. As a result, 28.3 g (yield 99%) of N-(biphenyl-4-yl)-N-{4-(2,4,6-triphenylphenyl)phenyl}amine was obtained as white solids.

### (Procedure 3)

A nitrogen-purged reaction vessel was charged with

| | |
|---|---|
| the resulting N-(biphenyl-4-yl)-N-{4-(2,4,6-triphenylphenyl)phenyl}amine | 6.0 g, |
| 3-bromo-9-phenyl-9H-carbazole | 3.9 g, |
| sodium tert-butoxide | 1.6 g, and |
| toluene | 60 ml, |

and a nitrogen gas was passed through the vessel for 45 minutes to obtain a mixture. To the mixture,

| | |
|---|---|
| palladium acetate | 0.1 g, and |
| tri-tert-butylphosphine | 0.2 g |

were added, followed by heating. The mixture was stirred for 3 hours at 104°C to obtain a reaction liquid. The reaction liquid was cooled to room temperature, and then methanol was added. Precipitated solids were collected by filtration. Toluene was added to the resulting solids, and the mixture was dissolved with heating. Then, silica gel was added, and the mixture was stirred and filtered. The filtrate was concentrated under reduced pressure. Methanol was added to precipitated solids, and the mixture was dispersed and washed. As a result, 6.7 g (yield 88%) of reference Compound 70 was obtained as white solids.

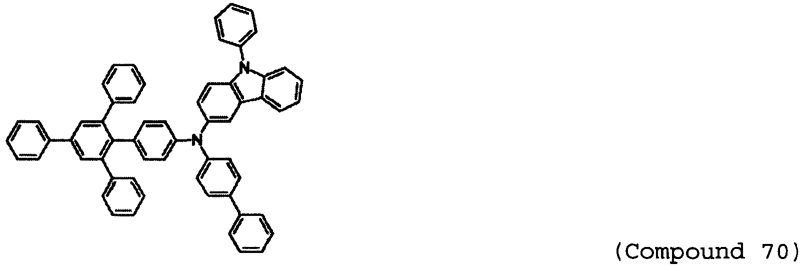

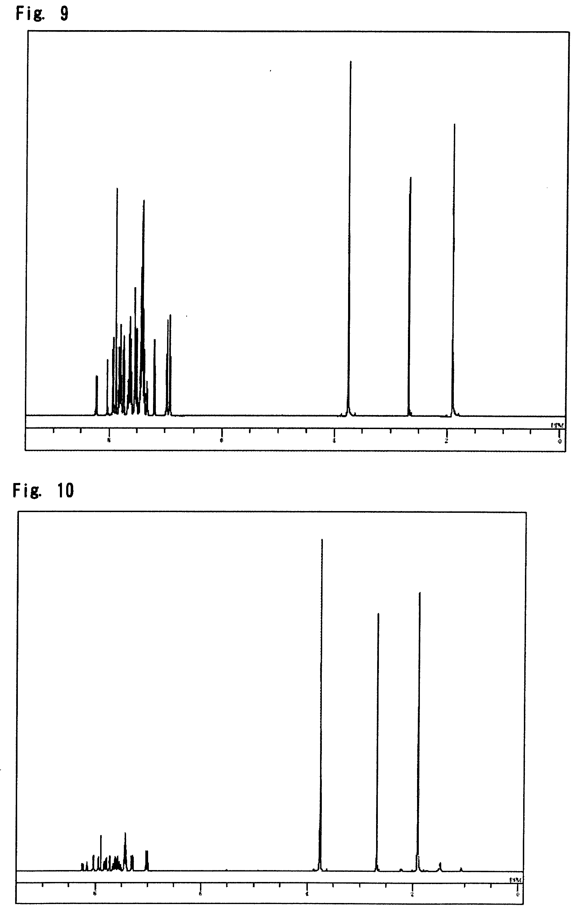

The structure of the resulting white solids was identified using NMR. The results of the ¹H-NMR measurement are shown in Fig. 9. In the ¹H-NMR (THF-d₈), the following signals of 42 hydrogens were detected.

| | |
|---|---|
| δ (ppm) | = 8.23 (1H) |
| | 8.04 (1H) |
| | 7.93 (2H) |
| | 7.88 (2H) |
| | 7.84-7.79 (4H) |
| | 7.75 (2H) |
| | 7.64 (5H) |
| | 7.55-7.50 (6H) |
| | 7.41 (12H) |
| | 7.33 (1H) |
| | 7.20 (2H) |
| | 6.98 (2H) |
| | 6.92 (2H) |

### <Example 10: Compound 94>

### Synthesis of N-(biphenyl-4-yl)-N-{4-(dibenzofuran-4-yl)phenyl}-N-[4-{(2,4,6-triphenyl)phenyl}phenyl]amine:

A nitrogen-purged reaction vessel was charged with

| | |
|---|---|
| 1-bromo-4-iodobenzene | 10.0 g, |
| 4-dibenzofuranylboronic acid | 7.9 g, |
| potassium carbonate | 9.8 g, |
| toluene | 80 ml, |
| ethanol | 20 ml, and |
| water | 40 ml. |

A nitrogen gas was passed through the vessel for 40 minutes to obtain a mixture. To this mixture, 0.8 g of tetrakis(triphenylphosphine)palladium was added, followed by heating, and the mixture was stirred for 6 hours at 74°C to obtain a reaction liquid. After the reaction liquid was cooled to room temperature, a liquid separating operation was performed with the addition of water to collect an organic layer. The organic layer was dehydrated over magnesium sulfate, and then concentrated under reduced pressure. Precipitated solids were collected by filtration. As a result, 5.6 g (yield 49%) of 4-(4-bromophenyl)dibenzofuran was obtained as yellow solids.

A nitrogen-purged reaction vessel was charged with

| | |
|---|---|
| the resulting 4-(4-bromophenyl)dibenzofuran | 3.9 g, |
| N-(biphenyl-4-yl)-N-{4-(2,4,6-triphenylphenyl)phenyl}amine obtained in Procedure 2 of Example 9 | 6.0 g, |
| sodium tert-butoxide | 1.6 g, and |
| toluene | 60 ml, |

and a nitrogen gas was passed through the vessel for 1 hour to obtain a mixture. To the mixture,

| | |
|---|---|
| palladium acetate | 0.1 g, and |
| tri-tert-butylphosphine | 0.2 g |

were added, followed by heating. The mixture was stirred for 3 hours at 103°C to obtain a reaction liquid. The reaction liquid was cooled to room temperature. Precipitated solids were collected by filtration. Monochlorobenzene was added to the resulting solids, and the mixture was dissolved with heating. Then, silica gel was added, and the mixture was stirred and hot-filtered. The filtrate was concentrated under reduced pressure. Methanol was added to precipitated solids, and the mixture was dispersed and washed. As a result, 7.5 g (yield 87%) of reference Compound 94 was obtained as white solids.

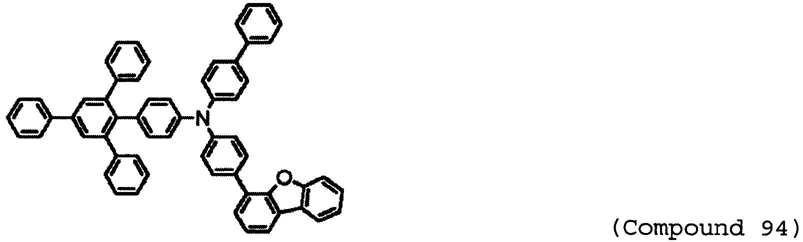

The structure of the resulting white solids was identified using NMR. The results of the ¹H-NMR measurement are shown in Fig. 10. In the ¹H-NMR (THF-d₈), the following signals of 41 hydrogens were detected.

| | |
|---|---|
| δ (ppm) | = 8.24 (1H) |
| | 8.15 (1H) |
| | 8.03 (2H) |
| | 7.95 (2H) |
| | 7.90 (2H) |
| | 7.83 (2H) |
| | 7.79 (2H) |
| | 7.73 (2H) |
| | 7.67-7.51 (8H) |
| | 7.43 (11H) |
| | 7.30 (4H) |
| | 7.02 (4H) |

### <Measurement of glass transition temperature>

The arylamine compounds of the present invention were measured for the glass transition temperature by a high sensitivity differential scanning calorimeter (DSC3100S, produced by Bruker AXS).

| Glass transition temperature (°C) | |
|---|---|
| Compound of Example 1 (Compound 2) | 110 |
| Compound of Example 2 (Compound 10) | 121 |
| Compound of Example 3 (Compound 41) | 121 |
| Compound of Example 4 (Compound 42) | 135 |
| Compound of Example 5 (Compound 57) | 128 |
| Compound of Example 6 (Compound 62) | 145 |
| Compound of Example 7 (Compound 91) | 143 |
| Compound of Example 8 (Compound 92) | 144 |
| Compound of Example 9 (Compound 70) | 134 |
| Compound of Example 10 (Compound 94) | 123 |

The arylamine compounds of the present invention had a glass transition temperature of 100° C or higher, demonstrating that they were stable in a thin film state.

### <Measurement of work function>

Using each of the compounds of the present invention, a vapor deposited film with a film thickness of 100 nm was prepared on an ITO substrate, and its work function was measured using an ionization potential measuring device (PYS-202, produced by Sumitomo Heavy Industries, Ltd.). Compounds 70 and 94 are reference compounds.

| | Work function (eV) |
|---|---|
| Compound of Example 1 (Compound 2) | 5.71 |
| Compound of Example 2 (Compound 10) | 5.67 |
| Compound of Example 3 (Compound 41) | 5.63 |
| Compound of Example 4 (Compound 42) | 5.55 |
| Compound of Example 5 (Compound 57) | 5.66 |
| Compound of Example 6 (Compound 62) | 5.67 |
| Compound of Example 7 (Compound 91) | 5.70 |
| Compound of Example 8 (Compound 92) | 5.63 |
| Compound of Example 9 (Compound 70) | 5.50 |
| Compound of Example 10 (Compound 94) | 5.70 |

The compounds of the present invention showed a suitable energy level as compared with a work function of 5.4 eV which an ordinary hole transport material such as NPD or TPD has. Thus, these compounds had satisfactory hole transport capability.

### <Device Example 1>

The hole injection layer 3, the hole transport layer 4, the electron blocking layer 5, the luminous layer 6, the electron transport layer 7, the electron injection layer 8, and the cathode (aluminum electrode) 9 were vapor-deposited in this order on an ITO electrode formed beforehand as the transparent anode 2 on the glass substrate 1 to prepare the organic EL device as shown in Fig. 11.

Concretely, the glass substrate 1 having a 50 nm thick ITO film formed thereon was washed with an organic solvent, and then the ITO surface was cleaned by UV/ozone treatment. Then, the glass substrate with the ITO electrode was placed in a vacuum deposition machine, and the pressure therein was reduced to 0.001 Pa or lower.

The hole injection layer 3 was formed. Concretely, a compound HIM-1 represented by the following structural formula was vapor-deposited so as to cover the transparent anode 2, whereby a layer with a film thickness of 5 nm was formed.

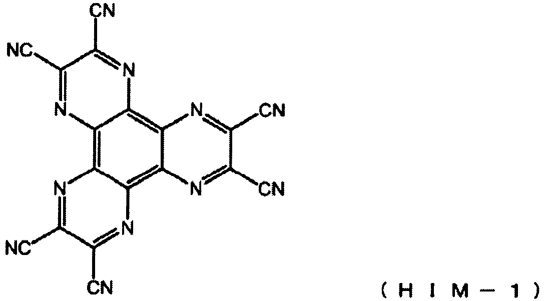

Then, the hole transport layer 4 was formed. Concretely, a compound HTM-1 represented by the following structural formula was vapor-deposited on the hole injection layer 3 to form a layer with a film thickness of 60 nm.

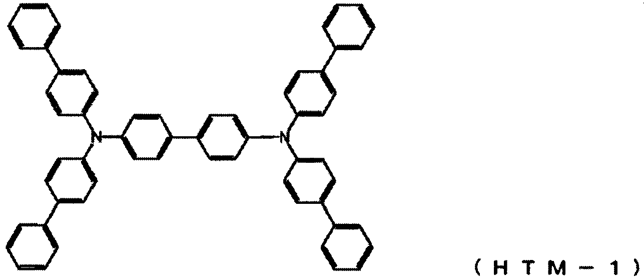

Then, the electron blocking layer 5 was formed. Concretely, the compound of Example 1 (Compound 2) was vapor-deposited on the hole transport layer 4 to form a layer with a film thickness of 5 nm.

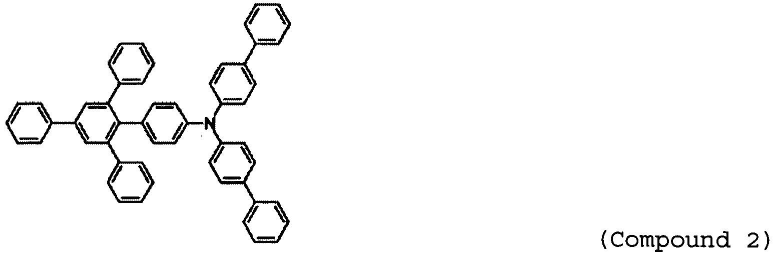

Then, the luminous layer 6 was formed. Concretely, a pyrene derivative EMD-1 of the following structural formula and an anthracene derivative EMH-1 of the following structural formula were binary vapor deposited on the electron blocking layer 5 at such vapor deposition rates that the vapor deposition rate ratio was EMD-1:EMH-1=5:95, whereby a layer was formed in a film thickness of 20 nm.

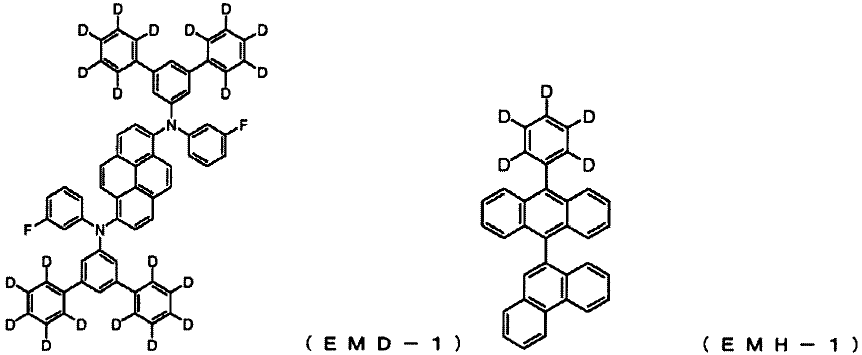

Then, the electron transport layer 7 was formed. Concretely, compounds ETM-1 and ETM-2 of the following structural formulas were binary vapor deposited on the luminous layer 6 at such vapor deposition rates that the vapor deposition rate ratio was ETM-1:ETM-2=50:50, whereby a layer was formed in a film thickness of 30 nm.

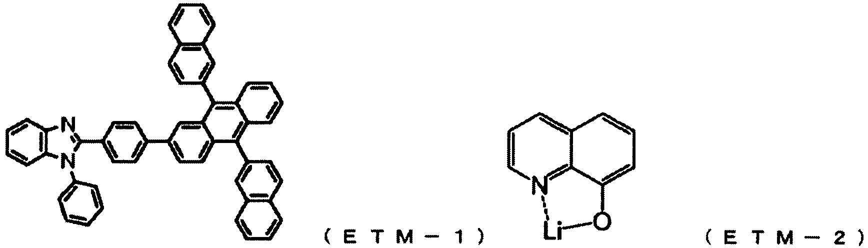

Then, the electron injection layer 8 was formed. Concretely, lithium fluoride was vapor deposited on the electron transport layer 7 to form the layer in a film thickness of 1 nm.

Finally, aluminum was vapor deposited to a film thickness of 100 nm to form the cathode 9.

The glass substrate having the organic films and the aluminum film formed thereon was moved into a glove box purged with dry nitrogen, and a sealing glass substrate was laminated thereto using a UV curing resin to produce an organic EL device.

### <Device Example 2>

An organic EL device was prepared under the same conditions as in Device Example 1, except that the compound of Example 2 (Compound 10) was used, instead of the compound of Example 1 (Compound 2), as the material for the electron blocking layer 5.

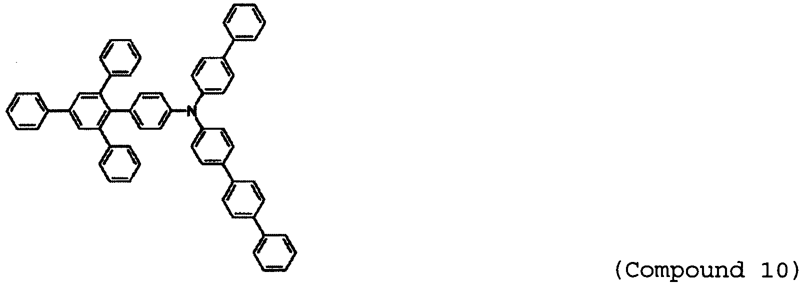

### <Device Example 3>

An organic EL device was prepared under the same conditions as in Device Example 1, except that the compound of Example 3 (Compound 41) was used, instead of the compound of Example 1 (Compound 2), as the material for the electron blocking layer 5.

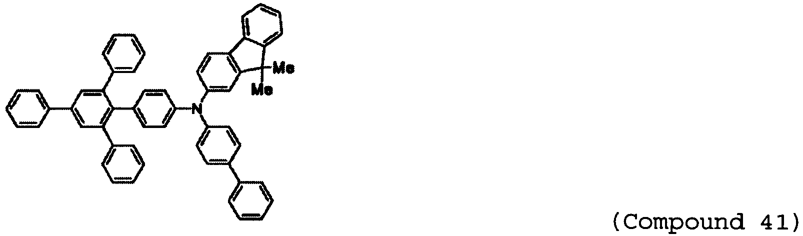

### <Device Example 4>

An organic EL device was prepared under the same conditions as in Device Example 1, except that the compound of Example 4 (Compound 42) was used, instead of the compound of Example 1 (Compound 2), as the material for the electron blocking layer 5.

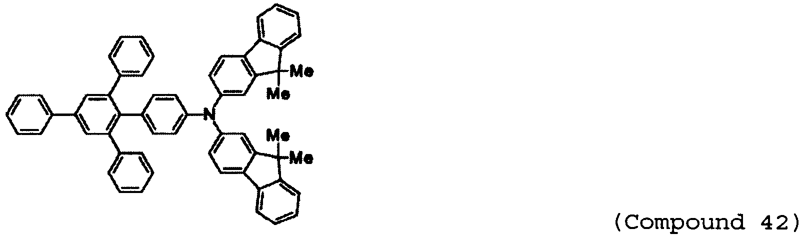

### <Device Example 5>

An organic EL device was prepared under the same conditions as in Device Example 1, except that the compound of Example 5 (Compound 57) was used, instead of the compound of Example 1 (Compound 2), as the material for the electron blocking layer 5.

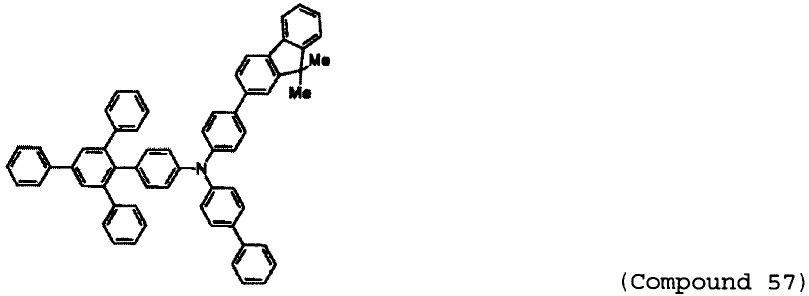

### <Device Example 6>

An organic EL device was prepared under the same conditions as in Device Example 1, except that the compound of Example 6 (Compound 62) was used, instead of the compound of Example 1 (Compound 2), as the material for the electron blocking layer 5.

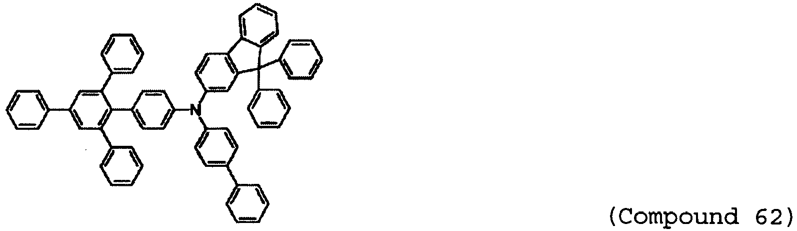

### <Device Example 7>

An organic EL device was prepared under the same conditions as in Device Example 1, except that the compound of Example 7 (Compound 91) was used, instead of the compound of Example 1 (Compound 2), as the material for the electron blocking layer 5.

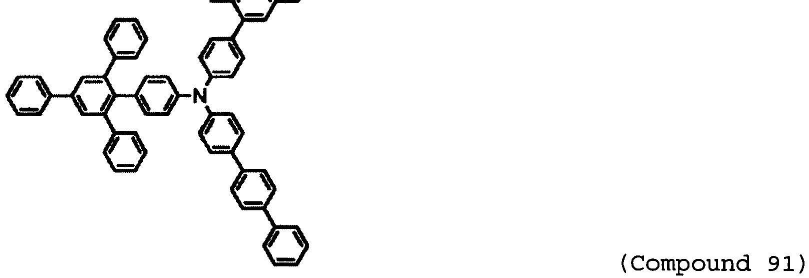

### <Device Example 8>

An organic EL device was prepared under the same conditions as in Device Example 1, except that the compound of Example 8 (Compound 92) was used, instead of the compound of Example 1 (Compound 2), as the material for the electron blocking layer 5.

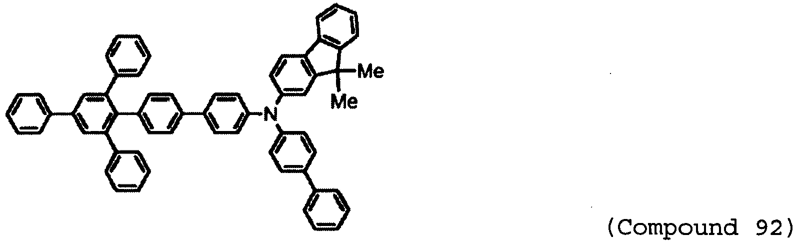

### <Device Example 9>

An organic EL device was prepared under the same conditions as in Device Example 1, except that the compound of Example 9 (reference compound 70) was used, instead of the compound of Example 1 (Compound 2), as the material for the electron blocking layer 5.

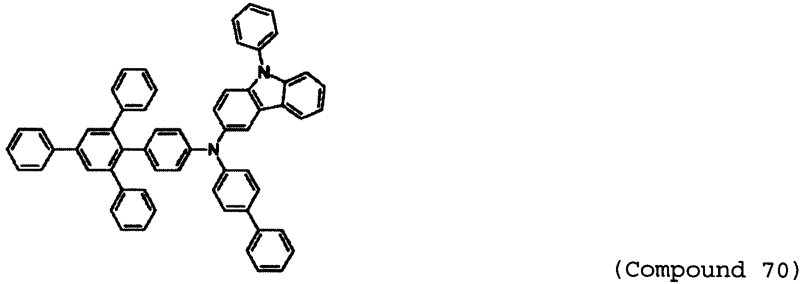

### <Device Example 10>

An organic EL device was prepared under the same conditions as in Device Example 1, except that the compound of Example 10 (reference compound 94) was used, instead of the compound of Example 1 (Compound 2), as the material for the electron blocking layer 5.

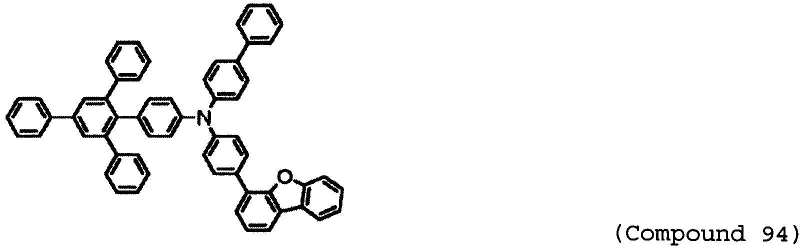

### <Device Comparative Example 1>

An organic EL device was prepared under the same conditions as in Device Example 1, except that a compound HTM-A of the following structural formula was used, instead of the compound of Example 1 (Compound 2), as the material for the electron blocking layer 5.

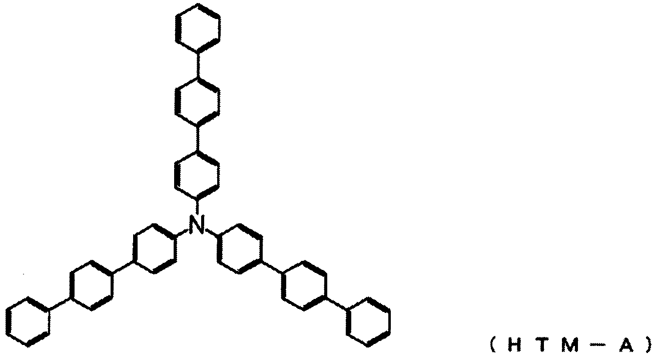

### <Device Comparative Example 2>

An organic EL device was prepared under the same conditions as in Device Example 1, except that a compound HTM-B of the following structural formula was used, instead of the compound of Example 1 (Compound 2), as the material for the electron blocking layer 5.

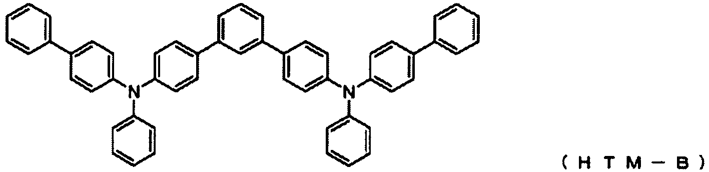

Each of the organic EL devices prepared in Device Examples 1 to 10 and Device Comparative Examples 1 to 2 was measured for the device lifetime. The results are shown in Table 1. The device lifetime was measured as the period of time until the emission luminance attenuated to 1900 cd/m² (corresponding to 95%, with the initial luminance taken as 100% : 95% attenuation) when constant current driving was performed, with the emission luminance at the start of light emission (initial luminance) being set at 2000 cd/m².

Each of the organic EL devices prepared in Device Examples 1 to 10 and Device Comparative Examples 1 to 2 was measured for the light emission characteristics when a direct current voltage was applied at normal temperature in the atmosphere. The results are shown in Table 1.

**Table 1**

| | Electron blocking layer | Voltage [V] (@10mA/cm²) | Luminance [cd/m²] (@10mA/cm²) | Luminous efficiency [cd/A] (@10mA/cm²) | Power efficiency [lm/W] (@10mA/cm²) | Device lifetime (hrs) 95% attenuation |
|---|---|---|---|---|---|---|
| Dev.Ex. 1 | Comp.2 | 3.85 | 835 | 8.35 | 6.82 | 194 |
| Dev.Ex. 2 | Comp.10 | 3.85 | 878 | 8.78 | 7.18 | 225 |
| Dev.Ex. 3 | Comp.41 | 3.77 | 821 | 8.21 | 6.84 | 209 |
| Dev.Ex. 4 | Comp.42 | 3.74 | 804 | 8.04 | 6.78 | 187 |
| Dev.Ex. 5 | Comp.57 | 3.84 | 913 | 9.13 | 7.48 | 184 |
| Dev.Ex. 6 | Comp.62 | 3.81 | 861 | 8.61 | 7.10 | 250 |
| Dev.Ex. 7 | Comp.91 | 3.87 | 923 | 9.23 | 7.51 | 201 |
| Dev.Ex. 8 | Comp.92 | 3.75 | 882 | 8.82 | 7.42 | 207 |
| Dev.Ex. 9 | Comp.70 | 3.65 | 822 | 8.22 | 7.08 | 183 |
| Dev.Ex. 10 | Comp.94 | 3.68 | 845 | 8.45 | 7.22 | 228 |
| Dev.Comp. Ex. 1 | HTM-A | 4.15 | 576 | 5.76 | 4.43 | 81 |
| Dev.Comp. Ex. 2 | HTM-B | 4.18 | 442 | 4.42 | 3.41 | 74 |

| | | | | | | |
|---|---|---|---|---|---|---|
| Dev. Ex.: Device Example Dev. Comp. Ex.: Device Comparative Example Comp.: Compound | | | | | | |

The driving voltage was 4.15 to 4.18V in Device Comparative Examples 1 to 2. On the other hand, it was 3.65 to 3.87V in Device Examples 1 to 10, demonstrating that the organic EL devices of the Device Examples were all drivable at low voltages.

The luminous efficiency was 4.42 to 5.76 cd/A in Device Comparative Examples 1 to 2, while it was as high as 8.04 to 9.23 cd/A in all of Device Examples 1 to 10.

The power efficiency was 3.41 to 4.43 lm/W in Device Comparative Examples 1 to 2, while it was as high as 6.78 to 7.51 lm/W in all of Device Examples 1 to 10.

The device lifetime was 74 to 81 hours in Device Comparative Examples 1 to 2. On the other hand, it was 183 to 250 hours in Device Examples 1 to 10, showing much longer lifetimes.

As the above results clearly shows, the organic EL devices using the arylamine compounds of the present invention were lower in driving voltage, higher in luminous efficiency, and longer in lifetime than the conventional organic EL devices.

### Industrial Applicability

The arylamine compound of the present invention has high hole transport capability, excels in electron blocking capability, and is stable in a thin film state. Thus, it is excellent as a compound for an organic EL device. When an organic EL device is prepared using this compound, a high luminous efficiency and a high power efficiency can be obtained, practical driving voltage can be lowered, and durability can be improved. Hence, the organic EL device of the present invention can be put to uses such as domestic electrical appliances and illumination.

### Explanations of Letters or Numerals

- 1: Glass substrate
- 2: Transparent anode
- 3: Hole injection layer
- 4: Hole transport layer
- 5: Electron blocking layer
- 6: Luminous layer
- 7: Electron Transport layer
- 8: Electron injection layer
- 9: Cathode

## Claims

1. An arylamine compound represented by the following general formula (1): 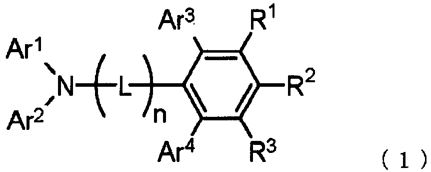 where
Ar¹ and Ar² may be the same or different, and each represent a phenyl group having a substituent or a fluorenyl group having a substituent, wherein the substituent for the phenyl group is selected from a phenyl group, a biphenylyl group, a terphenylyl group, a naphthyl group, a phenanthrenyl group or a fluorenyl group, and the substituent for the fluorenyl group is selected from a methyl group or a phenyl group,
Ar³ and Ar⁴ each represent an unsubstituted phenyl group,
L represents a 1,4-phenylene group,
R¹ and R³ each represent a hydrogen atom,
R² represents an unsubstituted phenyl group, and
n denotes an integer of 1 or 2.

2. An organic electroluminescent device having a pair of electrodes and at least one organic layer sandwiched therebetween, wherein
the arylamine compound according to claim 1 is used for the organic layer.

3. The organic electroluminescent device according to claim 2, wherein the organic layer is a hole transport layer.

4. The organic electroluminescent device according to claim 2, wherein the organic layer is an electron blocking layer.

5. The organic electroluminescent device according to claim 2, wherein the organic layer is a hole injection layer.

6. The organic electroluminescent device according to claim 2, wherein the organic layer is a luminous layer.

## Revendications

1. Composé arylamine représenté par la formule générale (1) suivante : 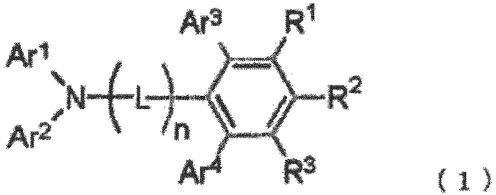 où
Ar¹ et Ar² peuvent être identiques ou différents et représentent chacun, un groupe phényle ayant un substituant ou un groupe fluorényle ayant un substituant, dans lequel le substituant du groupe phényle est choisi parmi un groupe phényle, un groupe biphénylyle, un groupe terphénylyle, un groupe naphtyle, un groupe phénanthrényle ou un groupe fluorényle, et le substituant du groupe fluorényle est choisi parmi un groupe méthyle ou un groupe phényle,
Ar³ et Ar⁴ représentent chacun un groupe phényle non substitué,
L représente un groupe 1,4-phénylène,
R¹ et R³ représentent chacun un atome d'hydrogène,
R² représente un groupe phényle non substitué, et
N représente un entier de valeur 1 ou 2.

2. Dispositif électroluminescent organique comportant une paire d'électrodes et au moins une couche organique intercalée entre elles, dans lequel le composé arylamine selon la revendication 1 est utilisé pour la couche organique.

3. Dispositif électroluminescent organique selon la revendication 2, dans lequel la couche organique est une couche de transport de trous.

4. Dispositif électroluminescent organique selon la revendication 2, dans lequel la couche organique est une couche de blocage des électrons.

5. Dispositif électroluminescent organique selon la revendication 2, dans lequel la couche organique est une couche d'injection de trous.

6. Dispositif électroluminescent organique selon la revendication 2, dans lequel la couche organique est une couche lumineuse.

## Patentansprüche

1. Arylaminverbindung, dargestellt durch die nachstehende allgemeine Formel (1): 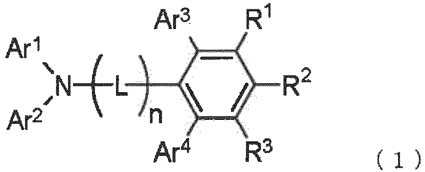 wobei
Ar¹ und Ar² gleich oder verschieden sein können und jeweils eine Phenylgruppe mit einem Substituenten oder eine Fluorenylgruppe mit einem Substituenten darstellen, wobei der Substituent für die Phenylgruppe ausgewählt ist aus einer Phenylgruppe, einer Biphenylylgruppe, einer Terphenylylgruppe, einer Naphthylgruppe, einer Phenanthrenylgruppe oder einer Fluorenylgruppe, und der Substituent für die Fluorenylgruppe ausgewählt ist aus einer Methylgruppe oder einer Phenylgruppe, Ar³ und Ar⁴ jeweils eine unsubstituierte Phenylgruppe darstellen,
L eine 1,4-Phenylengruppe darstellt,
R¹ und R³ jeweils ein Wasserstoffatom darstellen,
R² eine unsubstituierte Phenylgruppe darstellt, und
n eine ganze Zahl von 1 oder 2 bedeutet.

2. Organische Elektrolumineszenzvorrichtung mit einem Paar von Elektroden und mindestens einer dazwischen angeordneten organischen Schicht, wobei
die Arylaminverbindung nach Anspruch 1 für die organische Schicht verwendet wird.

3. Organische Elektrolumineszenzvorrichtung nach Anspruch 2, wobei die organische Schicht eine Lochtransportschicht ist.

4. Organische Elektrolumineszenzvorrichtung nach Anspruch 2, wobei die organische Schicht eine Elektronensperrschicht ist.

5. Organische Elektrolumineszenzvorrichtung nach Anspruch 2, wobei die organische Schicht eine Lochinjektionsschicht ist.

6. Organische Elektrolumineszenzvorrichtung nach Anspruch 2, wobei die organische Schicht eine Leuchtschicht ist.
